(19) Europäisches Patentamt
European Patent Office
Office européen des brevets

(11) **EP 4 505 961 A1**

(12) **EUROPEAN PATENT APPLICATION**
published in accordance with Art. 153(4) EPC

(43) Date of publication:
**12.02.2025 Bulletin 2025/07**

(21) Application number: **23802719.7**

(22) Date of filing: **28.04.2023**

(51) International Patent Classification (IPC):
*A61B 34/10* (2016.01)    *A61B 17/34* (2006.01)

(52) Cooperative Patent Classification (CPC):
**A61B 34/10;** A61B 17/34

(86) International application number:
**PCT/CN2023/091895**

(87) International publication number:
**WO 2023/216947 (16.11.2023 Gazette 2023/46)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **07.05.2022   CN 202210493274**
**30.06.2022   CN 202210764281**

(71) Applicant: **Wuhan United Imaging Surgical Co., Ltd.**
**Wuhan, Hubei 430073 (CN)**

(72) Inventors:
• **HE, Shaowen**
**Wuhan, Hubei 430073 (CN)**
• **LIAO, Mingzhe**
**Wuhan, Hubei 430073 (CN)**
• **ZHANG, Jing**
**Wuhan, Hubei 430206 (CN)**
• **FANG, Wei**
**Wuhan, Hubei 430206 (CN)**

(74) Representative: **Wang, Bo**
**Panovision IP**
**Ebersberger Straße 3**
**85570 Markt Schwaben (DE)**

(54) **MEDICAL IMAGE PROCESSING SYSTEM AND METHOD FOR INTERVENTIONAL OPERATION**

(57)    A medical image processing system and method for an interventional procedure are provided. The system includes a control system including at least one processor and at least one storage medium. The storage medium stores operating instructions. When executing the operating instructions, the at least one processor is directed to cause the system to perform operations including: obtaining a first medical image of a target object before the interventional procedure and a second medical image of the target object during the interventional procedure; registering the second medical image and the first medical image to obtain a registration result; and determining interventional procedure planning information of the target object at least based on the registration result, performing an interventional procedure risk assessment based on the interventional procedure planning information, and obtaining a risk assessment result corresponding to the interventional procedure planning information.

**200**

Obtaining a first medical image of a target object before an interventional procedure and a second medical image of the target object during the interventional procedure — 210

Registering the second medical image and the first medical image to obtain a registration result — 220

Determining interventional procedure planning information of the target object at least based on the registration result, performing an interventional procedure risk assessment based on interventional procedure planning information, and obtaining a risk assessment result corresponding to the interventional procedure planning information — 230

**FIG. 2**

EP 4 505 961 A1

## Description

## CROSS-REFERENCE TO RELATED APPLICATIONS

This application is a continuation of International Application No. PCT/CN2023/091895, filed on April 28, 2023, which claims priority to Chinese Application No. 202210493274.3, filed on May 7, 2022, and Chinese Application No. 202210764281.2, filed on June 30, 2022, the entire contents of each of which are hereby incorporated by reference.

## TECHNICAL FIELD

The present disclosure relates to the field of image processing technology, and in particular, to medical image processing methods, systems, and devices for interventional procedures, and computer storage media thereof.

## BACKGROUND

Computed tomography (CT) guided percutaneous interventional procedures are currently most commonly used for cancer diagnosis and treatment in clinical practice. Under real-time CT scanning, the physician master-slave controls a robot to perform a puncture, which greatly improves the efficiency and precision of the puncture, and reduces the radiation exposure dose on patients. It is urgent to assist physicians in controlling robots better to perform guided percutaneous interventional procedures.

Thus, the present disclosure provides a medical image processing system and method for interventional procedures to improve the efficiency of guided percutaneous interventional procedures.

## SUMMARY

One of the embodiments of the present disclosure provides a medical image processing system for an interventional procedure, comprising: a control system including at least one processor and at least one storage medium. The at least one storage medium storing operating instructions. When executing the operating instructions, the at least one processor is directed to cause the system to perform operations including: obtaining a first medical image of a target object before the interventional procedure and a second medical image of the target object during the interventional procedure; registering the second medical image and the first medical image to obtain a registration result; and determining interventional procedure planning information of the target object at least based on the registration result, performing an interventional procedure risk assessment based on the interventional procedure planning information, and obtaining a risk assessment result corresponding to the interventional procedure planning information.

One of the embodiments of the present disclosure provides a medical image processing method for an interventional procedure, comprising: obtaining a first medical image of a target object before the interventional procedure and a second medical image of the target object during the interventional procedure; registering the second medical image and the first medical image to obtain a registration result; and determining interventional procedure planning information of the target object at least based on the registration result, performing an interventional procedure risk assessment based on the interventional procedure planning information, and obtaining a risk assessment result corresponding to the interventional procedure planning information.

One of the embodiments of the present disclosure provides a medical image processing method for an interventional procedure, comprising: obtaining a mode of planning an interventional path; obtaining a preoperative enhanced image; obtaining a first medical image of a first target structure set by segmenting the first target structure set from the preoperative enhanced image; obtaining an intraoperative scanning image; obtaining a second medical image of a second target structure set by segmenting the second target structure set from the intraoperative scanning image, the first target structure set having an intersection with the second target structure set; registering the first medical image and the second medical image, determining a spatial position of a third target structure set in the interventional procedure, selecting elements of the third target structure set based on the mode of planning the interventional path; and obtaining the planned interventional path based on the spatial position of the third target structure set in the interventional procedure, and performing a procedure risk assessment based on the planned interventional path. At least one element in the third target structure set is included in the first target structure set and at least one element in the third target structure set is excluded from the second target structure set.

One of the embodiments of the present disclosure provides a guiding system for an interventional procedure, comprising a control system including at least one processor and at least one storage medium.

The at least one storage medium storing operating instructions. When executing the operating instructions, the at least one processor is directed to cause the system to perform operations including obtaining a first medical image, a second medical image, and a third medical image of a target object, respectively, at different times; registering the first medical image and the second medical image to obtain a fourth medical image, the fourth medical image including registered interventional procedure planning information; and mapping the fourth medical image to the third medical image to guide the interventional procedure.

One of the embodiments of the present disclosure provides a medical image processing device for an interventional procedure, comprising a processor. The processor is configured to execute operating instructions involved in the medical image processing system for the interventional procedure.

One of the embodiments of the present disclosure provides a non-transitory computer-readable storage medium storing computer instructions. When a computer reads the computer instructions in the storage medium, the computer performs the method as described in any of the embodiments.

**BRIEF DESCRIPTION OF THE DRAWINGS**

The present disclosure is further illustrated in terms of exemplary embodiments. These exemplary embodiments are described in detail with reference to according to the drawings. These embodiments are non-limiting exemplary embodiments, in which like reference numerals represent similar structures, and wherein:

FIG. 1 is a schematic diagram illustrating an exemplary application scenario for a medical image processing system for an interventional procedure according to some embodiments of the present disclosure;

FIG. 2 is a flowchart illustrating an exemplary process of medical image processing for an interventional procedure according to some embodiments of the present disclosure;

FIG. 3 is a flowchart illustrating an exemplary process of a guided interventional procedure according to some embodiments of the present disclosure;

FIG. 4 is a flowchart illustrating an exemplary process of medical image processing for an interventional procedure according to some embodiments of the present disclosure;

FIG. 5 is a flowchart illustrating an exemplary process of segmentation in medical image processing for an interventional procedure according to some embodiments of the present disclosure;

FIG. 6 is a flowchart illustrating an exemplary process for determining positioning information of an element mask according to some embodiments of the present disclosure;

FIG. 7 is a flowchart illustrating an exemplary process of a soft connected component analysis of an element mask according to some embodiments of the present disclosure;

FIG. 8 is a flowchart illustrating an exemplary process of a coarse segmentation for a soft connected component analysis of an element mask according to some embodiments of the present disclosure;

FIG. 9 is a flowchart illustrating an exemplary process of a fine segmentation of an element according to some embodiments of the present disclosure;

FIG. 10 is a schematic diagram illustrating determining positioning information of an element mask according to some embodiments of the present disclosure;

FIG. 11 is a schematic diagram illustrating determining positioning information of an element mask according to some embodiments of the present disclosure;

FIG. 12A is a schematic diagram illustrating determining a sliding direction based on positioning information of an element mask according to some embodiments of the present disclosure;

FIGs. 12B-12E are schematic diagrams illustrating a fine segmentation after a sliding window operation according to some embodiments of the present disclosure;

FIG. 13 is a diagram illustrating comparison of exemplary segmentation results according to some embodiments of the present disclosure;

FIG. 14 is a flowchart illustrating an exemplary process for registering a first medical image and a second medical image according to some embodiments of the present disclosure;

FIG. 15 is a flowchart illustrating an exemplary process for determining a registration deformation field according to some embodiments of the present disclosure;

FIG. 16 is a flowchart illustrating an exemplary process for determining a registration deformation field according to some embodiments of the present disclosure;

FIG. 17 is a schematic diagram illustrating obtaining a first medical image and a second medical image by segmentation according to some embodiments of the present disclosure;

FIG. 18 is a flowchart illustrating an exemplary process for determining intervention risk values of one or more elements of a third target structure set under a fast planning mode according to some embodiments of the present disclosure;

FIG. 19 is a flowchart illustrating an exemplary process for determining intervention risk values of one or more elements of a third target structure set under a precise planning mode according to some embodiments of the present disclosure;

FIG. 20 is a flowchart illustrating an exemplary process for detecting an image abnormality according to some embodiments of the present disclosure;

FIG. 21 is a flowchart illustrating an exemplary process for postoperative assessment according to some embodiments of the present disclosure;

FIG. 22 is a flowchart illustrating an exemplary process for postoperative assessment according to some embodiments of the present disclosure;

FIG. 23 is a flowchart illustrating an exemplary process for guiding an interventional procedure according to some embodiments of the present disclosure;

FIG. 24 is a schematic diagram illustrating an exemplary process for guiding an interventional procedure according to some embodiments of the present disclosure;

FIG. 25 is another schematic diagram illustrating an exemplary process for guiding an interventional procedure according to some other embodiments of the present disclosure;

FIG. 26 is a schematic diagram illustrating exemplary modules of a medical image processing system for an interventional procedure according to some embodiments of the present disclosure; and

FIG. 27 is a schematic diagram illustrating an exemplary user interface for guiding a puncture procedure according to some embodiments of the present disclosure.

## DETAILED DESCRIPTION

To more clearly illustrate the technical solutions related to the embodiments of the present disclosure, a brief introduction of the drawings referred to the description of the embodiments is provided below. Obviously, the drawings described below are only some examples or embodiments of the present disclosure. Those having ordinary skills in the art, without further creative efforts, may apply the present disclosure to other similar scenarios according to these drawings. Unless obviously obtained from the context or the context illustrates otherwise, the same numeral in the drawings refers to the same structure or operation.

It should be understood that "system", "device", "unit" and/or "module" as used herein is a manner used to distinguish different components, elements, parts, sections, or assemblies at different levels. However, if other words serve the same purpose, the words may be replaced by other expressions.

As shown in the present disclosure and claims, the words "one", "a", "a kind" and/or "the" are not especially singular but may include the plural unless the context expressly suggests otherwise. In general, the terms "comprise," "comprises," "comprising," "include," "includes," and/or "including," merely prompt to include operations and elements that have been clearly identified, and these operations and elements do not constitute an exclusive listing. The methods or devices may also include other operations or elements.

The flowcharts used in the present disclosure illustrate operations that systems implement according to some embodiments of the present disclosure. It should be understood that the previous or subsequent operations may not be accurately implemented in order. Instead, each step may be processed in reverse order or simultaneously. Meanwhile, other operations may also be added to these processes, or a certain step or several steps may be removed from these processes.

Some terms or concepts involved in the embodiments of the present disclosure are explained as follows.

An interventional procedure, which is also referred to as interventional therapy, is a minimally invasive therapeutic procedure utilizing contemporary high-technology means. Specifically, it may be performed under the guidance of medical scanning devices or medical imaging devices, whereby special catheters, guide wires, and other precision instruments are introduced into the human body, for diagnosis and localized treatment of pathologies in the body. In some embodiments of the present disclosure, the interventional procedure is also referred to as a puncture or a puncture procedure, which may be used interchangeably without prejudice to confusion.

Preoperative planning, short for preoperative planning for the interventional procedure, is an essential assisting part of the interventional procedure. The accuracy of preoperative planning directly affects the accuracy of an interventional path in the interventional procedure, which in turn affects the outcome of the interventional procedure.

A target object, which may also be referred to as a scanning object, may include a whole or part of a biological object and/or a non-biological object involved in a scanning process. For example, the target object (the scanning object) may be an animate or inanimate organic and/or inorganic substance, such as the head, ears, nose, mouth, neck, chest, abdomen, liver, gallbladder, pancreas, spleen, kidneys, spine, or the like.

The implementation of the interventional procedure is often very complex. To implement the procedure more smoothly, in the related technology, computed tomography (CT) guided percutaneous interventional procedure is used in the clinical diagnosis and treatment of cancer. Under real-time CT scanning, the physician master-slave controls a robot for puncture, thus the efficiency and accuracy of puncture are greatly improved, and the radiation irradiation dose on patients is reduced. However, due to the limitations of radiation dose, imaging time, etc., the range of real-time CT scanning is small, which affects the real-time puncture field. If the lesion is large, or the entry point is far away from the target point, or the user wants to completely observe the status of the entire target organ during the real-time puncture, the scanning range needs to be expanded. However, expanding the scanning range of real-time CT scanning may result in a layer thickness of a CT image being too large to recognize the detailed information inside the target organ. Especially when the lesion is small, it may not be possible to show detailed information of the lesion in the real-time scanned image.

In addition, there may be a lack of precision in preoperative planning in the relevant techniques, and workflows implemented based on inaccurate planning are also relatively homogenous, which has a poor risk-averse property, thus leading to poor surgical outcomes. On the other hand, the related technology lacks intraoperative puncture path guidance and target (lesion) display during intraoperative navigation, and the computation of real-time simulation planning is too complex and time-consuming, making it difficult to apply to clinical scenarios.

Therefore, embodiments of the present disclosure provide better methods to help physicians perform the interventional procedure better.

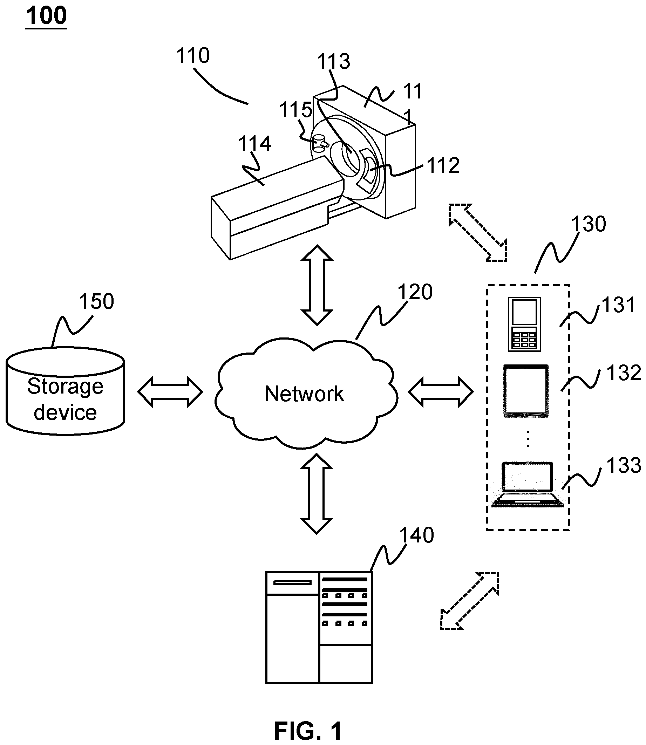

FIG. 1 is a schematic diagram illustrating an exemplary application scenario for a medical image processing system for an interventional procedure according to some embodiments of the present disclosure.

In some embodiments, a medical image processing system 100 may be applied to a plurality of interventional procedures or interventional treatments. In some embodiments, the interventional procedures or interventional treatments may include a cardiovascular interventional procedure, an oncology interventional procedure, an obstetrics and gynecology interventional procedure, a skeletal muscle interventional procedure, or any other feasible interventional procedures, such as neurological interventional surgery, etc. In some embodiments, the interventional procedure or interventional treatments may also include percutaneous transluminal biopsy, coronary angiography, thrombolysis, stenting, or any other feasible interventional procedure, such as an ablative procedure, etc.

As shown in FIG. 1, the medical image processing system 100 may include a medical scanning device 110, a network 120, one or more terminals 130, a processing device 140, and a storage device 150. The connections between components in the medical image processing system 100 may be variable. For example, the medical scanning device 110 may be connected to the processing device 140 via the network 120. As another example, the medical scanning device 110 may be directly connected to the processing device 140, as indicated by the dashed bi-directional arrow connecting the medical scanning device 110 and the processing device 140. As yet another example, the storage device 150 may be connected to the processing device 140 either directly or via the network 120. Merely by way of example, the terminal 130 may be connected directly to the processing device 140 (as shown by the dashed arrow connecting the terminal 130 and the processing device 140), or it may be connected to the processing device 140 via the network 120.

The medical scanning device 110 may be configured to scan a scanning object using high-energy rays (e.g., X-rays, gamma rays, etc.) to collect scanning data related to the scanning object. The scanning data may be used to generate one or more images of the scanning object. In some embodiments, the medical scanning device 110 may include an ultrasound imaging (US) device, a computed tomography (CT) scanner, a digital radiography (DR) scanner (e.g., mobile digital radiography), a digital subtraction angiography (DSA) scanner, a dynamic spatial reconstruction (DSR) scanner, an X-ray microscope scanner, a multimodal scanner, or the like, or a combination thereof. In some embodiments, the multimodal scanner may include a computed tomography-positron emission tomography (CT-PET) scanner and a computed tomography-magnetic resonance imaging (CT-MRI) scanner. The scanning object may be biological or non-biological. Merely by way of example, the scanning object may include patients, man-made objects (e.g., man-made phantoms), or the like. As another example, the scanning object may include specific parts, organs, and/or tissues of the patient.

In some embodiments, the medical scanning device 110 may include a gantry 111, a detector 112, a detection region 113, a table 114, and a radiation source 115. The gantry 111 may support the detector 112 and the radiation source 115. The scanning object may be placed on the table 114 for scanning. The radiation source 115 may emit radiation rays to the scanning object. The detector 112 may detect the radiation rays (e.g., X-rays) emitted from the radiation source 115. In some embodiments, the detector 112 may include one or more detector units. The detector units may include a scintillation detector (e.g., a cesium iodide detector), a gas detector, or the like. The detector unit may include a single line detector and/or a multi-line detector.

The network 120 may include any suitable network capable of facilitating the exchange of information and/or data of the medical image processing system 100. In some embodiments, one or more components of the medical image processing system 100 (e.g., the medical scanning device 110, the terminal 130, the processing device 140, the storage device 150) may exchange information and/or data with each other over the network 120. For example, the processing device 140 may obtain image data from the medical scanning device 110 via the network 120. As another example, the processing device 140 may obtain user instructions from the terminal 130 via the network 120.

The network 120 may be and/or include a public network (e.g., the Internet), a private network (e.g., a local area network (LAN), a wide area network (WAN), etc.), a wired network (e.g., an Ethernet network, a wireless network (e.g., an 802.11 network, a Wi-Fi network, etc.), a cellular network (e.g., a long-term evolution (LTE) network), a frame relay network, a virtual private network ( "VPN"), a satellite network, a telephone network, a router, a hub, a switch, a server computer, and/or any combination thereof. In some embodiments, network 120 may include one or more network access points. For example, the network 120 may include wired and/or wireless network access points, such as a base station and/or an Internet exchange point, through which one or more components of the medical image processing system 100 may be connect to network 120 to exchange data and/or information.

The terminal 130 may include a mobile device 131, a tablet computer 132, a laptop computer 133, etc., or any combination thereof. In some embodiments, the mobile device 131 may include a smart home device, a wearable device, a mobile device, a virtual reality device, an augmented reality device, etc., or any combination thereof. In some embodiments, the terminal 130 may be part of the processing device 140.

The processing device 140 may process data and/or information obtained from the medical scanning device 110, the terminal 130, and/or the storage device 150. For example, the processing device 140 may obtain data obtained by the medical scanning device 110 and use the data for imaging to generate medical images (e.g., a preoperative enhanced image and an intraoperative scanning image) and segment the medical images to generate segmentation result data, e.g., a first segmentation image (a first medical image), a second segmentation image (a second medical image), a spatial position of the intra-surgical vessels and the lesion, an registration map, etc. As another example, the processing device 140 may obtain the medical images, planning mode data (e.g., precise planning mode data and fast planning mode data), and/or scanning protocols from the terminal 130. As another example, the processing device 140 may obtain data (e.g., a segmentation and registration result, an intervention risk value, a predetermined weight, a weighted risk value, a cumulative risk value, an image abnormality type, an image abnormality degree, or the like) obtained by the medical scanning device 110 and utilize the data for processing to generate an interventional path and/or a prompt message.

In some embodiments, the processing device 140 may be a single server or a group of servers. The group of servers may be centralized or distributed. In some embodiments, the processing device 140 may be local or remote. For example, the processing device 140 may access information and/or data stored in the medical scanning device 110, the terminal 130, and/or the storage device 150 via the network 120. As another example, the processing device 140 may be directly connected to the medical scanning device 110, the terminal 130, and/or the storage device 150 to access the stored information and/or data. In some embodiments, processing device 140 may be implemented on a cloud platform.

The storage device 150 may store data, instructions, and/or any other information. In some embodiments, the storage device 150 may store data obtained from the medical scanning device 110, the terminal 130, and/or the processing device 140. For example, the storage device 150 may store medical image data (e.g., a preoperative enhanced image, an intraoperative scanning image, the first medical image, the second medical image, etc.) and/or positioning information data obtained from the medical scanning device 110. As another example, the storage device 150 may store the medical image and/or a scanning protocol input from the terminal 130. As another example, the storage device 150 may store data generated by the processing device 140 (e.g., the medical image data, organ mask data, positioning information data, result data obtained after fine segmentation, the spatial positions of the intra-surgical vessels and the lesion, the registration map, etc.). As yet another example, the storage device 150 may store data generated by the processing device 140 (e.g., the segmentation and registration result, the intervention risk value, the predetermined weight, the weighted risk value, the cumulative risk value, the image abnormality type, the image abnormality degree, the interventional path, and/or the alert message, etc.).

In some embodiments, the storage device 150 may store data and/or instructions that the processing device 140 may execute or use to execute the exemplary methods described in the present disclosure. In some embodiments, the storage device 150 may include a mass storage device, a removable storage device, a volatile read-write memory, a read-only memory (ROM), or the like, or any combination thereof. An exemplary mass storage device may include a disk, an optical disk, a solid-state drive, or the like. In some embodiments, the storage device 150 may be implemented on a cloud platform.

In some embodiments, the storage device 150 may be connected to the network 120 to communicate with one or more other components (e.g., the processing device 140 and the terminal 130) in the medical image processing system 100. One or more components of the medical image processing system 100 may access data or instructions stored in the storage device 150 via the network 120. In some embodiments, the storage device 150 may be directly connected to or in communication with one or more other components of the medical image processing system 100 (e.g., the processing device 140 and the terminal 130). In some embodiments, the storage device 150 may be part of the processing device 140.

The description of the medical image processing system 100 is intended to be illustrative and is not intended to limit the scope of the present disclosure. Numerous substitutions, modifications, and variations will be apparent to those of ordinary skill in the art. It will be appreciated that to those skill in the art, knowing the principle of the system, it may be possible, without departing from this principle, to make any combination of modules, or to form sub-systems to be connected to other modules.

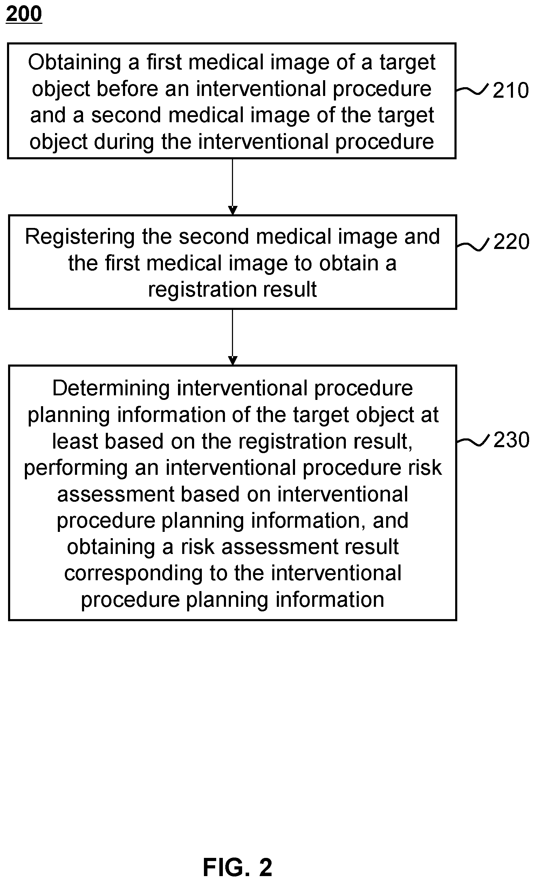

FIG. 2 is a flowchart illustrating an exemplary process of medical image processing for an interventional procedure according to some embodiments of the present disclosure. In some embodiments, the process 200 may be implemented by the medical image processing system 100 or a processing device (e.g., the processing device 140) for an interventional procedure. In some embodiments, the method of processing medical images for interventional procedures may be implemented by a control system including a medical image processing system. The control system may include at least one processor and at least one storage medium. The storage medium may store operating instructions that cause the processor to execute process 200.

In 210, a first medical image of the target object may be obtained before the interventional procedure and a second medical image of the target object may be obtained during the interventional procedure.

The first medical image refers to a medical image of the target object obtained before performing the interventional procedure. The second medical image refers to a medical image of the target object obtained during the interventional procedure.

In some embodiments, the first medical image and/or the second medical image may include a computed tomography (CT) image, a positron emission tomography-computed tomography (PET-CT) image, an ultrasound (US) image, or a magnetic resonance (MR) image, etc.

In some embodiments, the first medical image, the second medical image of the target object may be obtained at the same respiratory amplitude point or at a similar respiratory amplitude point that does not affect puncture accuracy. For example, the first medical image may be obtained when the target object is at a first respiratory amplitude point before the interventional procedure, and the second medical image may be obtained when the target object is at a second respiratory amplitude point during the interventional procedure and before the puncture is performed. A deviation of second respiratory amplitude point from the first respiratory amplitude point is less than a predetermined value. The time period during the interventional procedure and before the puncture may be a time period during the preparation for the interventional procedure when the puncture needle has not yet been inserted in the puncture, which may be a time period in which whether the puncture needle enters into the body of the target object is a critical time point.

The respiratory amplitude refers to a physical quantity that reflects changes in air volume during respiration. The respiratory amplitude point refers to a time point at which one is at a certain respiratory amplitude, e.g., at the end of inhalation, at the end of exhalation, at a certain intermediate state of inhalation, at a certain intermediate state of exhalation, etc. In some embodiments, the respiratory amplitude point of the obtained image (e.g., the first medical image and the second medical image) may be determined based on needs, experience, and/or user habits. For example, when doing a lung puncture, the lesion is compressed less in the inspiratory state, and the image may be obtained at the end of inspiration.

In some embodiments, before the interventional procedure, before the puncture is performed during the interventional procedure, or during the puncture, the target object may perform self-adjustment on his or her own (or under the guidance of a technician) to a certain respiratory amplitude point (e.g., the end of inhalation), and the first medical image and the second medical image may be obtained at the respiratory amplitude point by a medical scanning device (e.g., the medical scanning device 110).

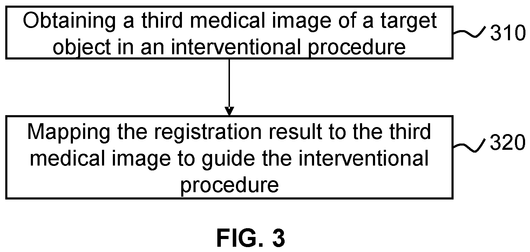

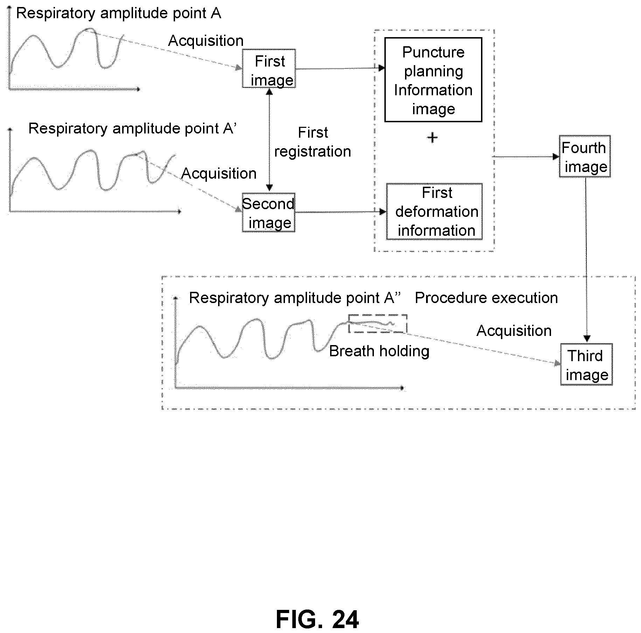

In some embodiments, the processing device may cause the first medical image and the second medical image to be obtained when the target object is at the same, or nearly the same respiratory amplitude point via a respiratory gating device. For example, as shown in FIG. 24, the respiratory gating device may obtain a respiratory amplitude point A where the target object is located when obtaining the first medical image. During the interventional procedure and before the puncture, the respiratory gating device may monitor the breathing of the target object and cause the medical scanning device to obtain the second medical image when the target object is at a respiratory amplitude point A'. In some embodiments, the respiratory amplitude of the target object is monitored by the respiratory gating device during the interventional procedure, and a third medical image may also be obtained using the medical scanning device when the target object adjusts his or her breathing to the respiratory amplitude point A". Descriptions regarding the third medical image may be found in FIG. 3 and related descriptions thereof. In some embodiments, the breathing amplitude of the target object is monitored during the breath-holding of the target object (i.e., keeping the breathing amplitude near the point A"). A prompt may also be issued to the user to assist the user in adjusting the breathing when the breathing amplitude of the target object deviates significantly from the point A".

Obtaining the first medical image, the second medical image, and the third medical image at the same or nearly the same respiratory amplitude point allows for less movement of the organs and tissues between images caused by respiratory motion, which is conducive to improving the accuracy of preoperative planning.

In some embodiments, the preset value may be set based on demand and/or experience, e.g., by setting the preset value to 1%, 5%, 7%, 10%, or the like. As shown in FIG. 24, the first medical image is obtained at a first respiratory amplitude point A, the second medical image is obtained at a second respiratory amplitude point A' at which the deviation from the first respiratory amplitude point A is less than the predetermined value, and the third medical image is obtained at a third respiratory amplitude point A" point where the deviation from the first respiratory amplitude point A and/or the second respiratory amplitude point A' is less than the predetermined value.

In some embodiments, the obtained first medical image and second medical image may be medical images that have undergone certain processing, e.g., segmentation processing.

In some embodiments, the processing device may obtain the preoperative enhanced image and segment a first target structure set of the preoperative enhanced image to obtain the first medical image of the first target structure set. The processing device may obtain the intraoperative scanning image and segment a second target structure set of the intraoperative scanning image to obtain the second medical image of the second target structure set.

The preoperative enhanced image refers to an image of the target object (e.g., a patient, etc.) scanned by the medical scanning device (e.g., the medical scanning device 110) after the injection of a contrast agent into the target object before the procedure. In some embodiments, the preoperative enhanced image may include a CT image, a PET-CT image, a US image, an MR image, etc. In some embodiments, the processing device may obtain the preoperative enhanced image of the target object from the medical scanning device 110, or may read from the terminal, database, and storage device to obtain the preoperative enhanced image of the target object. In some embodiments, the preoperative enhanced image may also be obtained by any other feasible means, e.g., the preoperative enhanced image may be obtained from a cloud-based server and/or a healthcare system (e.g., a healthcare system center at a hospital, etc.) via a network (e.g., the network 120), which is not limited by embodiments of the present disclosure.

The intraoperative scanning image refers to an image of the target object obtained after the target object has been scanned by the medical scanning device during the procedure. In some embodiments, the intraoperative scanning image may include a CT image, a PET-CT image, a US image, an MR image, etc. In some embodiments, the intraoperative scanning image may be a real-time scanning image. In some embodiments, the intraoperative scanning image, which may also be referred to as a preoperative plain scanning image or an intraoperative plain scanning image, is a scanning image that is taken during procedure preparation and before the procedure (i.e., before the actual needle insertion).

In some embodiments, the first target structure set of the preoperative enhanced image may include blood vessels within a target organ. In some embodiments, the first target structure set of the preoperative enhanced image may include the target organ and a lesion in addition to blood vessels within the target organ. In some embodiments, the target organ may include the brain, the lungs, the liver, the spleen, the kidneys, or any other possible organ tissue, such as the thyroid gland. The first medical image refers to a medical image of the first target structure set (e.g., the target organ, the blood vessels within the target organ, the lesion in the preoperative enhanced image) obtained by segmenting the preoperative enhanced image.

In some embodiments, the region or organ included in the second target structure set of the intraoperative scanning image may be determined based on a mode of planning the interventional path. The interventional path refers to a path through which instruments used in the interventional procedure are introduced into the body of the target object. The mode of planning the interventional path may include a precise planning mode and a fast planning mode. In some embodiments, the precise planning mode or the fast planning mode may be a path planning mode for segmenting the intraoperative scanning image. In some embodiments, the precise planning mode may include a fine segmentation mode. In some embodiments, the fast planning mode may include a fast segmentation mode. In some embodiments, the second target structure set includes regions or organs that may be different when the modes of planning the interventional path are different. For example, under the fast planning mode, the second target structure set may include a non-interventional region. As another example, under the precise planning mode, the second target structure set may include all the vital organs in the intraoperative scanning image. The vital organs refer to organs that need to be avoided by the planned interventional path during the interventional procedure, for example, the liver, the kidneys, the external blood vessels of the target organ, etc. In some embodiments, the second target structure set may include target organs and lesions in addition to all the vital organs in the non-interventional region or the intraoperative scanning image. The second medical image refers to a medical image of the second target structure set (e.g., non-interventional region or vital organs, the target organ, the lesion) in the procedure obtained by segmenting the intraoperative scanning image.

In some embodiments, the first target structure set has an intersection with the second target structure set. For example, when the first target structure set includes blood vessels within the target organ and the target organ, and the second target structure set includes the non-interventional region (or all the vital organs), the intersection of the first target structure set and the second target structure set is the target organ. As another example, when the first target structure set includes blood vessels within the target organ, the target organ, and the lesion, and the second target structure set includes the non-interventional region (or all the vital organs), the target organ, and the lesion, the intersection of the first target structure set and the second target structure set is the target organ and the lesion.

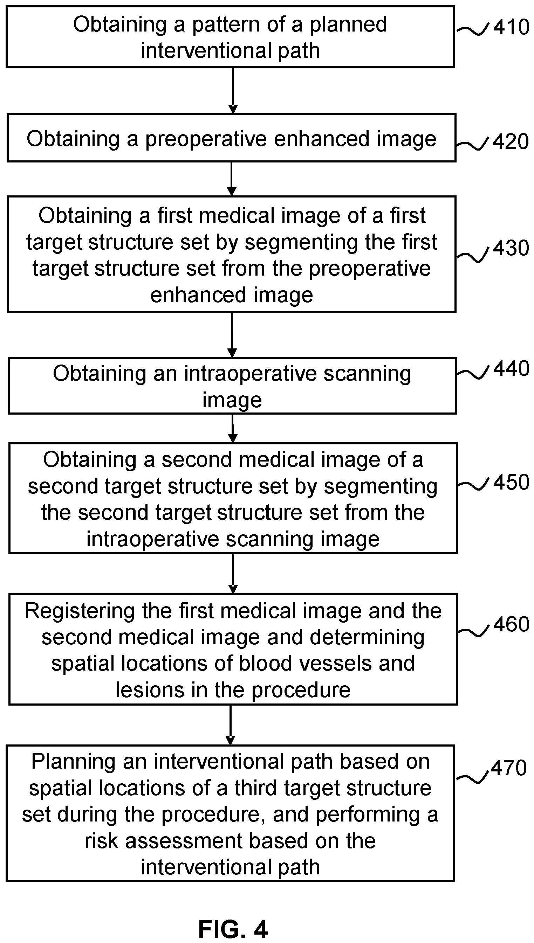

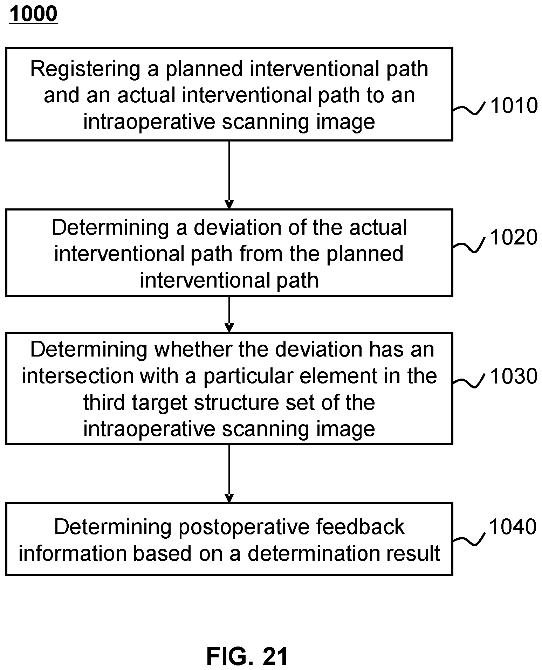

More descriptions regarding the first medical image, the second medical image, and the third medical image may be found in FIGs. 4 and 21 and related descriptions thereof and will not be repeated here.

In 220, the second medical image and the first medical image may be registered to obtain a registration result.

The process of registering refers to a process of matching and superimposing different images obtained at different times or under different conditions. For example, the process of registering may be an image processing operation that achieves spatial and anatomical position consistency between corresponding points of the first medical image and the second medical image through spatial transformation. Multidimensional information included in different images may be comprehensively reflected by the registration.

The registration result refers to an image obtained after registering the second medical image and the first medical image. In some embodiments, the registration result may also be referred to as a fourth medical image.

In some embodiments, the registration result may include a spatial position of the third target structure set during the procedure. The third target structure set refers to a full set of structures obtained by registering the first medical image and the second medical image, e.g., elements in the third target structure set may include elements in both the first target structure set and the second target structure set. In some embodiments, the third target structure set may include the target organ, blood vessels within the target organ, the lesion, and other regions or organs (e.g., the non-interventional region, all the vital organs). In some embodiments, under the fast segmentation mode, the other regions/organs may refer to non-interventional regions; under the fine segmentation mode, the other regions or organs may refer to all the vital organs. In some embodiments, at least one element in the third target structure set is included in the first target structure set, and at least one element in the third target structure set is not included in the second target structure set. For example, when the first target structure set includes the blood vessels within the target organ, the target organ, and the lesion, and the second target structure set includes the non-interventional region (or all the vital organs), the target organ, and the lesion, then the blood vessels within the target organ may be included in the first target structure set and not included in the second target structure set. In some embodiments, the elements of the third target structure set may be determined based on the mode of planning the interventional path. The mode of planning the interventional path may include a precise planning mode and a fast planning mode. Descriptions regarding the mode of planning the interventional path may be found in the description of operation 410 in FIG. 4 hereinafter.

In some embodiments, the processing device may obtain a registration deformation field by matching the second medical image and the first medical image, and the registration deformation field may be used to reflect spatial position variations of the first medical image and the second medical image. The registration result may be obtained by superimposing the second medical image and the first medical image based on the registration deformation field. The registration deformation field may be used to reflect the spatial position variations of the first medical image and the second medical image. Based on the position deformation field, the second medical image and the first medical image may be superimposed, and the registration result may be obtained. After transforming the spatial position based on the registration deformation field, the transformed intraoperative scanning image may be made to be identical to the preoperative enhanced image in the spatial position and the anatomical position.

In some embodiments, the processing device may register the first medical image and the second medical image using a non-rigid registration algorithm based on features, grayscales, or the like, such as a Demons-based non-rigid registration algorithm. In some embodiments, the processing device 140 may further register the first medical image and the second medical image using a deep learning-based non-rigid registration algorithm, to improve the real-time performance of the registration.

Exemplarily, a process for registering the first medical image and the second medical image may be shown in the following embodiment.

First, the processing device may obtain an interventional procedure planning information image based on the first medical image.

The interventional procedure planning information image refers to an image including interventional procedure planning information. In some embodiments, the interventional procedure planning information may include at least one of high-risk tissue information, a planned puncture path, and lesion position information. A high-risk tissue refers to an organ and/or tissue such as large blood vessels, bones, etc., that may have adverse effects on the target object and/or procedure process if punctured. In some embodiments, different high-risk tissues may be set up according to individual circumstances of different target objects. For example, the liver of a patient with low hepatic function is used as a danger zone, and other lesions in the target object's body are used as danger zones. The planned puncture path refers to a planned route of travel of the puncture instrument. The planned puncture path information may include an entry point, a target point, a puncture angle, a puncture depth, a path length, tissues and/or organs passed by the path, or the like. The lesion position information may include coordinates, a depth, a volume, margins, etc., of the lesion (or a center of the lesion) in the body coordinate system.

In some embodiments, the processing device or a relevant person (e.g., a doctor) may perform processing such as segmentation of the first medical image to obtain the interventional procedure planning information. For example, various tissues or organs, such as blood vessels, skin, bones, organs, tissues to be punctured, or the like, may be segmented. As another example, the segmented tissues or organs may be classified into a lesion region, a penetrable region, a high-risk tissue, or the like. As yet another example, a planned puncture path may be determined based on the lesion region, the penetrable region, the high-risk tissue, or the like. In some embodiments, the processing device or the relevant person (e.g., the doctor) may annotate the interventional procedure planning information on the first medical image to obtain an interventional procedure planning information image.

Secondly, the processing device may perform a first registration on the first medical image and the second medical image to obtain first deformation information. The first deformation information refers to information about a morphological change of an image element (e.g., a pixel or voxel) in the second medical image relative to a corresponding image element in the first medical image. For example, geometric change information, projection change information, or the like. The first deformation information may be represented by a first deformation matrix. Exemplarily, the first deformation matrix may include a deformation matrix in the x-direction, a deformation matrix in the y-direction, and a deformation matrix in the z-direction. An element in each deformation matrix corresponds to a unit region (e.g., 1 pixel point, a 1 mm×1 mm image region, 1 voxel point, a 1 mm×1 mm×1 mm image region, etc.) of the second medical image, and the value of the element is deformation information of the unit region in the x-axis direction, y-axis direction, or z-axis direction. In some embodiments, the processing device may perform first registration, through the Demons-based non-rigid registration algorithm, a geometric correction algorithm, the deep-learning-based non-rigid registration algorithm, etc., on the first medical image and the second medical image to obtain the first deformation information.

Finally, the processing device may actuate the first deformation information on the interventional procedure planning information image to obtain the registration result. The interventional procedure planning information in the registration result refers to interventional procedure planning information after the first registration. Exemplarily, the processing device may apply the first deformation matrix to the interventional procedure planning information image, i.e., cause the interventional procedure planning information image and the interventional procedure planning information (the high-risk tissue information, the planned puncture path, the lesion position information, etc.) to produce a morphological change corresponding to the first deformation information, thereby obtaining the registration result. The interventional procedure planning information in the registration result is interventional procedure planning information after the first registration.

The first medical image is obtained before the interventional procedure, with relatively ample time for acquisition and image processing, and a scanning range of the first medical image is relatively large area and slices are thicker, e.g., including a large number of slices encompassing all of the relevant tissues and/or organs. Planning the puncture path on a more comprehensively informative first medical image facilitates the accuracy of subsequent interventional procedure guidance. The second medical image is obtained during the interventional procedure and before the puncture, with a relatively tight time for acquisition and image processing, and the scanning range of the second medical image is relatively small and slices are thin, which, for example, may include only 4 to 10 slices encompassing surrounding the needle tip. The registration result (e.g., the fourth medical image) obtained by registering the first medical image and the second medical image may include the registered interventional procedure planning information. The time to achieve a highly accurate registration is generally long, taking from a few seconds to about a dozen seconds. Therefore, by preemptively registering the first medical image with the second medical image with high precision during the interventional procedure and before the puncture, the computational stress after the start of the puncture may be avoided or minimized, enabling the actual interventional procedure to be performed immediately or performed within a shorter time period after obtaining real-time images, reducing the duration of the puncture.

In 230, interventional procedure planning information of the target object may be determined at least based on the registration result, an interventional procedure risk assessment may be performed based on the interventional procedure planning information, and a risk assessment result corresponding to the interventional procedure planning information may be obtained.

In some embodiments, the interventional procedure planning information may also be referred to as puncture planning information.

As described above, the registration result may include annotated interventional procedure planning information, thus the processing device may determine the interventional procedure planning information of the target object based directly on the registration result.

The risk assessment refers to a process of analyzing and determining risks that may arise during the execution of puncture. The risk assessment result may be a summary of the risk assessment. The spatial position of elements in the registration result (e.g., the target organ, the lesion, the blood vessels within the target organ, the non-interventional region, all the vital organs) provides a comprehensive and accurate reflection of the current status of the target object (e.g., the patient). The interventional planning information reduces the risk of the procedure while enabling the surgical instruments (e.g., puncture needles) to reach the lesion while avoiding blood vessels, the non-interventional region, and/or all the vital organs in the target organ, and risk assessment of the interventional planning information further reduces the risk of the interventional procedure.

In some embodiments, performing the risk assessment based on the interventional procedure planning information may include determining intervention risk values of at least a part of the elements of the registration result and performing the risk assessment based on the intervention risk values. For example, the processing device may determine intervention risk values of one or more elements of the third target structure set and perform the risk assessment based on the intervention risk values, each of the intervention risk values corresponding to one of the one or more elements.

An intervention risk value may indicate a level of intervention risk of the element. In some embodiments, the higher the intervention risk value, the higher the level of intervention risk, i.e., the higher the intervention risk. For example, a region of an element with an intervention risk value of 8 is riskier to intervene than a region of an element with an intervention risk value of 6.

In some embodiments, the selection of the elements of the third target structure set may be determined based on the mode of planning the interventional path. In some embodiments, the elements of the third target structure set used to determine the risk assessment of the interventional path may be different when the modes of planning the interventional path are different. For example, in the fast planning mode, the elements of the third target structure set used to determine the risk assessment of the interventional path may include blood vessels within the target organ and non-interventional regions. As another example, in the precise planning mode, the elements of the third target structure set used to determine the risk assessment of the interventional path may include blood vessels within the target organ and all the vital organs.

In some embodiments, the processing device may determine whether the interventional path in the interventional procedure planning information crosses a predetermined element in the third target structure set. In response to a determination that the planned interventional path in the interventional procedure planning information crosses the predetermined element in the third target structure set, the intervention risk value of a predetermined risk object in the third target structure set may be determined.

In some embodiments, the third target structure centralized predetermined element may refer to a target organ. The predetermined risk object in the third target structure set refers to a blood vessel within the target organ. It will be appreciated that the predetermined risk object may be included in one or more elements of the third target structure set used to perform the risk assessment.

In some embodiments, if the interventional path crosses a target organ of the third target structure set, the blood vessels within the target organ and the non-interventional region may be at a certain risk relative to the interventional path under the fast planning mode, and intervention risk values of the blood vessels within the target organ and the non-interventional region relative to the interventional path may need to be calculated. In the fast planning mode, the blood vessels within the target organ and external vital organs or tissues may be at a certain risk relative to the interventional path, and intervention risk values of the blood vessels within the target organ and the external vital organs or tissues relative to the interventional path may need to be calculated. In some embodiments, if the interventional path does not cross the target organ in the third target structure set, the blood vessels within the target organ may be not at risk relative to the interventional path, and there may be no need to consider the effect of the vessels within the target organ on the interventional path (or the blood vessels within the target organ having intervention risk values of zero). Therefore, if the interventional path does not cross the target organ of the third target structure set, in the fast planning mode, only the intervention risk value of the non-interventional region relative to the interventional path may need to be calculated, and in the precise planning mode, only the intervention risk values of the vital organs or tissues outside the target organ relative to the interventional path may be calculated. Based on the fact whether the interventional path crosses the target organ, by determining the intervention risk values of the elements to be calculated in different planning modes, the risk assessment may be more rationally performed on the interventional path.

In some embodiments, the manner of determining whether the interventional path crosses the target organ may include obtaining an intersection between a target organ mask and the interventional path. If the intersection is not an empty set, the interventional path crosses the target organ; otherwise, the interventional path does not cross the target organ.

More descriptions regarding the risk assessment of the interventional procedure may be found in the related description of FIG. 3.

In some embodiments, in response to the risk assessment result of the interventional procedure planning information satisfying a predetermined condition, the processing device may guide the interventional procedure based on the interventional procedure planning information that satisfies the predetermined condition. The predetermined condition may be that the intervention risk value is less than a predetermined threshold value. For example, assuming that the predetermined threshold value is 7, the predetermined condition is considered not to be satisfied when the intervention risk value is 8, and the predetermined condition is considered satisfied when the intervention risk value is 6. Guiding the intervention procedure based on the interventional procedure planning information that satisfies the predetermined conditions may be assisting in guiding the movement of the surgical instruments within the target object according to the interventional procedure planning information that satisfies the predetermined condition, to avoid the target organ within the blood vessels, non-interventional region, and/or all the vital organs in the target organ to reach the lesion smoothly and realize treatment of the patient.

FIG. 3 is a flowchart illustrating an exemplary process of a guided interventional procedure according to some embodiments of the present disclosure.

In 310, a third medical image of the target object may be obtained in the interventional procedure.

The third medical image may be a real-time image during the interventional procedure. In some embodiments, the third medical image is obtained during the interventional procedure. The process of performing the interventional procedure may include entering the needle from the skin, following the interventional path into a target region, completing the operation in the target region, and exiting the needle.

In some embodiments, the third medical image may be obtained by a computed tomography (CT) scanning device.

In some embodiments, the third medical image may be obtained by an imaging device different from the one that obtains the first medical image and the second medical image. For example, the first medical image and the second medical image may be obtained by an imaging device in an imaging room, and the third medical image may be obtained by an imaging device in an operating room. In some embodiments, image parameters (e.g., an image range, accuracy, contrast, a gray scale, a gradient, etc.) of the first medical image, the second medical image, and the third medical image may be the same or different. For example, a scanning range of the first medical image may be greater than scanning ranges of the second medical image and the third medical image, or the accuracy of the second medical image and the third medical image may be higher than the first medical image.

In some embodiments, the third medical image may be obtained at the same respiratory amplitude point of the target object when obtaining the first medical image and the second medical image, or at a similar respiratory amplitude point that does not affect the accuracy of the puncture. As shown in FIG. 24, for example, the first medical image is obtained at the first respiratory amplitude point A, the second medical image is obtained at the second respiratory amplitude point A' with a deviation from the first respiratory amplitude point A being less than a predetermined value, and the third medical image is obtained at a third respiratory amplitude point A" point with a deviation from the first respiratory amplitude point A and/or the second respiratory amplitude point A' being less than the predetermined value.

During the interventional procedure, the target object may perform a self-adjustment (or be adjusted under the guidance of a technician) to a certain respiratory amplitude point (e.g., at the end of inhalation), and the medical scanning device may obtain the third medical image at the respiratory amplitude point.

In 320, the registration result may be mapped to the third medical image to guide the interventional procedure.

In some embodiments, the processing device may map the fourth medical image to the third medical image to guide the interventional procedure using homography transformation, affine transformation, alpha channel transformation, or the like. For example, the user may perform the mapped puncture path under the guide of the mapped third medical image and avoid high-risk regions such as blood vessels, and progressively puncture toward the mapped lesion.

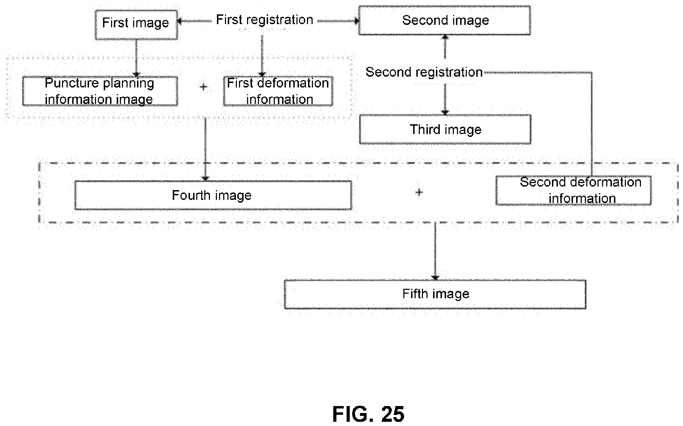

In some embodiments, if the breathing of the target object is not monitored by a respiratory gating device, the second medical image and the third medical image may be obtained when the target object is at different respiratory amplitude points, the organs and/or tissues in the images may move, and the processing device may perform a second registration on the second medical image and the third medical image. As shown in FIG. 25, in some embodiments, second deformation information may be obtained by performing the second registration on the second medical image and the third medical image.

The second deformation information refers to morphology change information of an image element in the third medical image relative to a corresponding image element in the second medical image. For example, geometric change information, projection change information, etc. The second deformation information may be represented by a second deformation matrix. Exemplarily, the second deformation matrix may include a deformation matrix in the x-direction, a deformation matrix in the y-direction, and a deformation matrix in the z-direction. An element in each deformation matrix corresponds to a unit region (e.g., 1 pixel point, a $1\ mm \times 1\ mm$ image region, 1 voxel point, a $1\ mm \times 1\ mm \times 1\ mm$ image region, etc.) of the third medical image, and the value of the element is deformation information of the unit region in the x-axis direction, y-axis direction, or z-axis direction. In some embodiments, the processing device may perform the second registration, through a Demons-based non-rigid registration algorithm, a geometric correction algorithm, a deep-learning-based non-rigid registration algorithm, etc., on the second medical image and the third medical image to obtain the second deformation information.

In some embodiments, the processing device may apply the second deformation information to the registration result (e.g., the fourth medical image) to obtain a fifth medical image. The fifth medical image may include interventional procedure planning information obtained after the second registration. For example, the processing device may apply the second deformation matrix to the registration result so that the interventional procedure planning information (e.g., high-risk tissue information, planned puncture path, lesion position information, etc.) contained in the registration result obtained after the first registration produces a morphological change corresponding to the second deformation information, thereby obtaining the fifth medical image.

In some embodiments, the processing device may map the fifth medical image to the third medical image. For example, the processing device may map the fifth medical image to the third medical image by homography transformation, affine transformation, alpha channel transformation, or the like.

Because the second medical image and the third medical image are both data at slice-less layers, the fast registration has a small computational cost, and the registration may be realized in a shorter time period after obtaining the third medical image during the procedure, reducing the risk of procedure.

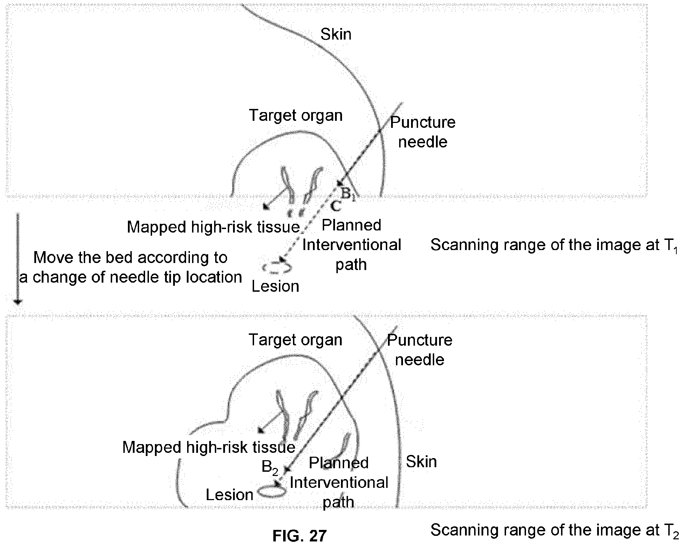

In some embodiments, when the interventional procedure is performed, the processing device may display the registration result (e.g., the fourth medical image) or image information in the fifth medical image that is located outside the display range of the third medical image. For example, the processing device may display the registration result or other tissues and/or organs in the fifth medical image, etc. As another example, the processing device may display a lesion outside the display range of the third medical image at the T1 moment, as illustrated in FIG. 27.

The processing device may display the planned path information of the corresponding interventional procedure outside the display range of the third medical image. For example, if the lesion is outside the scanning range, as shown in FIG. 27, the planned path from point C to the lesion is displayed.

The processing device may display the image information within and outside the display range of the third medical image in different ways. For example, different background colors may be set inside and outside the display range, RGB images may be displayed inside the display range and grayscale images may be displayed outside the display range, lines inside the display range (e.g., planning paths) may be displayed as solid lines, and lines outside the display range (e.g., planning paths) may be displayed as dashed lines (or solid lines of a different color), etc.

The scanning range of the third medical imaging scans may be relatively small due to limitations such as a radiation dose, an imaging time, and other limitations that affect real-time puncture vision. By presenting information outside of the display range, puncture planning information mapped outside the scanning range of the third medical image scan may be supplemented into the real-time image of the interventional procedure to broad the scale of the user viewing the planning information during the puncture procedure, to obtain more useful information about the interventional procedure. In particular, when a lesion is not within the scanning range of the real-time image (e.g., the third medical image) at the start of the puncture procedure, a lesion that is displayed outside the scanning range may give the doctor a clear target for the puncture, making the procedure more likely to be successful.

In some embodiments, the processing device may recognize a puncture needle tip position based on the third medical image. The processing device may extract a puncture needle in the third medical image obtained by a semi-automatic threshold segmentation algorithm or a fully automated deep learning algorithm, which in turn may obtain the needle tip position. For example, as shown in FIG. 27, the processing device may recognize a puncture needle tip at the T1 moment at the point B1.

The processing device may move the target object, a bed carrying the target object, or a detector for capturing the third medical image, based on the needle tip position, such that the needle tip is located in a central region of the display range of the third medical image. For example, as shown in FIG. 27, at the moment T1, the needle tip is punctured toward the lesion along the planned path, and the processing device 140 may anticipate the needle tip position at the moment T2 at the point B2, and move the bed in the time period of T1 to T2 according to the change of the needle tip position so that the scanning range of the third medical image is moved downward so that the needle tip is located in the central region of the display range of the third medical image.

By moving the target object, the bed carrying the target object, or the detector for capturing the third medical image, the scanning range is updated in real time, keeping the needle tip in the central region of the display range of the third medical image, which may highlight the information around the needle tip, and the traveling process of the needle tip may be tracked more accurately, which is conducive to improving the efficiency of the procedure and reducing the risk of the procedure.

In some embodiments, in order to further minimize the risk of procedure, the control system may also cause the one or more processors to perform the following operations.

An abnormal image recognition model is obtained. A real-time medical image of the target object during the procedure is obtained. Based on real-time medical image, a real-time risk probability of the target object is obtained using the abnormal image recognition model. When the risk probability reaches a predetermined threshold, the doctor is reminded in real-time.

The abnormal image recognition model may be a machine learning model for recognizing whether there is an abnormal condition in an image. The abnormal condition may include bleeding, puncture needles crossing high-risk tissue, or the like. In some embodiments, the abnormal image recognition model may include a deep neural network model, a convolutional neural network model, or the like. The abnormal image recognition model may be obtained by obtaining historical real-time medical image(s) during historical interventional procedure(s) and using the historical real-time medical image(s) as training sample(s), manually labeling the historical real-time medical image(s) based on a determination of whether abnormal condition(s) have actually occurred and using determination result(s) as label(s), and training an initial abnormal image recognition model using the training sample(s). The training may be realized using a plurality of model training algorithms, such as, a gradient descent algorithm, etc., which are not limited in the present disclosure.

The real-time medical image refers to an image obtained during the interventional procedure performed on the target object. The real-time medical image may be obtained through real-time scanning by the medical scanning device, or by other manners, such as reading an image of the procedure from a storage device, a database, which may be the second medical image, the third medical image, and the intraoperative scanning image.

In some embodiments, the processing device may input the real-time medical image to the abnormal image recognition model, and the abnormal image recognition model may output a real-time risk probability of the target object. The real-time risk probability is a probability of a risk occurrence when continuing the current procedure.

The predetermined threshold (e.g., a real-time probability threshold), e.g., 0.8, 0.6, 0.5, etc., may be set in advance, and the settings may be performed manually or in other ways, which are not limited by the present disclosure.

In some embodiments, the control system may cause one or more processors to perform the following operations.

Characteristic information of the target object is obtained. A risk of an interventional procedure of the target object in a next time period is predicted using a risk prediction model based on the real-time medical image, an actual progress of a current interventional procedure and an actual puncture path.

The characteristic information of the target object refers to data that may reflect a personal characteristic of the target object, such as age, gender, height, weight, body fat percentage, blood pressure, the presence of an underlying disease, and the category of the underlying disease. The actual progress of the current intervention may include a procedure execution time, a procedure completion degree (e.g., 5%, 10%, 20%, etc.), and an entry depth of the puncture needle. The actual puncture path may be the same as or different from an interventional procedure planning path. For example, certain adjustments may be made during the interventional procedure based on the interventional procedure planning path according to the actual situation. In some embodiments, the actual puncture path may be obtained based on the real-time medical image by image recognition processing, e.g., by segmenting an actual needle entry path of the puncture needle from the real-time medical image and determining the actual puncture path based on a segmentation result.

The processing device may input the characteristic information of the target object, the real-time medical image, the actual progress of the current interventional procedure, and the actual puncture path into the risk prediction model for processing, and the risk prediction model may output the interventional procedure risk of the target object in the next time period. The next time period may be 1 minute later, 2 minutes later, or 5 minutes later. In some embodiments, the next time period may include a plurality of time points, and the risk prediction model may simultaneously output the probability of occurring a procedure risk at each time point.

In some embodiments, the risk prediction model may include a deep neural network model, a convolutional neural network model, or the like, or other combinations of models. The risk prediction model may be obtained by obtaining historical characteristic information of the target object, historical real-time medical image(s), historical actual progress(es) of the current interventional procedure, and historical actual puncture path(s) and using them as training samples, manually labeling the training samples based on a determination of whether the abnormal condition has actually occurred and using the determination result(s) as label(s), and training an initial risk prediction model using the training samples. The training may be realized by a plurality of model training algorithms, such as, a gradient descent algorithm, etc., which are not limited in the present disclosure.

In some embodiments, the control system may also cause one or more processors to perform the following operations.

The processing device may obtain actual interventional procedure information of a plurality of time periods; obtain interventional deviation information by comparing actual interventional procedure information of each time period and the corresponding interventional procedure planning information; perform clustering based on the interventional deviation information and display a clustering result.

The actual interventional procedure information may include an actual interventional procedure path, actual lesion position information in the interventional procedure, etc. The actual lesion position information refers to lesion position information obtained based on the real-time medical image in the interventional procedure. The actual interventional procedure path may be a path that the puncture needle passes through from the beginning of the interventional procedure, as the puncture needle enters into the target object, to the current moment. With the passing of time, actual interventional procedure paths of a plurality of time periods may be obtained.

The interventional deviation information refers to a difference between the actual interventional procedure path in the actual interventional procedure information and the interventional procedure planning path and a difference between the actual lesion position information and the lesion position information in the interventional procedure planning information. In some embodiments, the processing device may obtain the interventional deviation information in a plurality of ways such as direct comparison, calculation of variance, etc., and the interventional deviation information may be represented in an image or a matrix, etc. For example, the actual interventional procedure path and the interventional procedure planning path may be displayed on a same image, and the difference between the two (e.g., a deviation distance between the paths, etc.) may be labeled. In addition, the actual lesion position and the lesion position in the interventional procedure planning information may also be displayed on one image at the same time, and the difference between the two may be labeled.

The clustering refers to a process of aggregating the interventional deviation information of a plurality of time periods. For example, interventional deviation information in the plurality of time periods corresponding to the same position (e.g., a position where a deviation between the actual interventional procedure path and the interventional procedure planning path is 0 in a first time period, the deviation is 1 in the second time period, the deviation is 0.5 in the third time period, etc.) may be clustered. Through clustering, deviation information at different time points may be integrated and displayed to the doctor, which makes it convenient for the doctor to understand the key parts of the interventional procedure that are likely to have deviations or the key times of occurring deviations and then make targeted adjustments to the subsequent interventional procedure.

FIG. 4 is a flowchart illustrating an exemplary process of medical image processing for an interventional procedure according to some embodiments of the present disclosure.

In 410, a mode of planning an interventional path may be obtained.

In some embodiments, an interventional path is a pathway through which instruments used in an interventional procedure are introduced into the body. The mode of planning the interventional path may include a precise planning mode and a fast planning mode. In some embodiments, the precise planning mode or the fast planning mode may be a path planning mode for segmenting scanning images in a procedure. In some embodiments, the precise planning mode may include a fine segmentation mode. In some embodiments, the fast planning mode may include a fast segmentation mode.

In some embodiments, the mode of planning the interventional path may be obtained. In some embodiments, the mode of planning the interventional path may be obtained from the medical scanning device 110. In some embodiments, the mode of planning the interventional path may be obtained from the terminal 130, the processing device 140, and the storage device 150.

In 420, a preoperative enhanced image may be obtained.

In some embodiments, the processing device may obtain the preoperative enhanced image of the scanning object, such as a PET-CT image, etc., from the medical scanning device 110. In some embodiments, the processing device may obtain the preoperative enhanced image of the scanning object, such as a US image, etc., from the terminal 130, the processing device 140, and the storage device 150.

In 430, a first medical image of a first target structure set may be obtained by segmenting the first target structure set from the preoperative enhanced image.

In some embodiments, the first medical image may also be referred to as a first segmentation image.

In 440, an intraoperative scanning image may be obtained.

In some embodiments, the processing device may obtain the intraoperative scanning image of the scanning object, such as a PET-CT image, etc., from the medical scanning device 110. In some embodiments, the processing device may obtain the intraoperative scanning image of the scanning object, such as a US image, etc., from the terminal 130, the processing device 140, and the storage device 150.

In 450, a second medical image of a second target structure set may be obtained by segmenting the second target structure set from the intraoperative scanning image. In some embodiments, the second medical image may also be referred to as a second segmentation image.

In some embodiments, segmentation of a target organ of the intraoperative scanning image by a processing device may be implemented in the following manner. The second target structure set from the intraoperative scanned image is segmented according to a planning mode. In some embodiments, a fourth target structure set of the intraoperative scanning image may be segmented according to the fast segmentation mode and/or the fine segmentation mode.

In some embodiments, the fourth target structure set may be a part of the second target structure set, e.g., non-interventional regions, all vital organs outside the target organ. The fourth target structure set includes different regions or organs in different planning modes. In some embodiments, in the fast segmentation mode, the fourth target structure set may include the non-interventional region. In some embodiments, in the fine segmentation mode, the fourth target structure set may include a predetermined vital organ.

In some embodiments, in the fast segmentation mode, region positioning calculations may be performed on the intraoperative scanning image, and segmentation and extraction may be performed on the non-interventional region.

The non-interventional region refers to a region that the planned interventional path needs to avoid during the interventional procedure. In some embodiments, the non-interventional region may include a non-puncturable region, a non-importable or placeable region, and a non-injectable region.

In some embodiments, post-processing may be performed on regions other than the non-interventional region and the target organ to ensure that there is no hollow region in an intermediate region between the non-interventional region and the target organ. The hollow region refers to a background region enclosed by the boundary formed by connected foreground pixels. In some embodiments, the non-interventional region may be obtained by subtracting the target organ and an interventional region from the abdominal (or thoracic) region. After subtracting the target organ and the interventional region from the abdominal (or thoracic) region to obtain the non-interventional region, there may be a hollow region in an intermediate region of the target organ and the non-interventional region, which belongs neither to the target organ nor to the non-interventional region. At this time, a post-processing operation may be performed on the hollow region to complete the hollow region, i.e., the hollow region after the post-processing operation may be regarded as the non-interventional region. In some embodiments, the post-processing may include an erosion operation and an expansion operation. In some embodiments, the erosion operation and the expansion operation may be implemented based on convolution processing of the intraoperative scanning image and a filter. In some embodiments, the erosion operation may be calculating a local minimum based on a predetermined erosion range after the filter is convolved with the intraoperative scanning image, such that the contour of the intraoperative scanning image is reduced to a desired range and the intraoperative scanning displays that a target highlighted region of an initial image is reduced by a certain range. In some embodiments, the expansion operation may be calculating a local maximum based on the predetermined erosion range after the filter is convolved with the intraoperative scanning, such that the contour of the intraoperative scanning image is expanded to a desired range, and a target highlighted region in the intraoperative scanning image displays that the initial image is reduced by a certain range.

In some embodiments, in the fast segmentation mode, the segmentation extraction of the non-interventional region may be performed after performing region positioning calculations on the intraoperative scanning image. In some embodiments, a vascular mask within the target organ may be determined based on a segmentation mask and a vascular mask of the target organ of the intraoperative scanning image. It should be noted that in the fast segmentation mode, only the blood vessels inside the target organ need to be segmented; and in the fine segmentation mode, both the blood vessels inside the target organ and other blood vessels outside the target organ may be segmented.

A mask, such as an organ mask, may be a pixel-level categorization label. Taking an abdominal medical image as an example, the mask indicates the categorization of individual pixels in the medical image, e.g., it may be categorized into background, liver, spleen, kidney, etc. The aggregated region of a specific category is represented by a corresponding label value. For example, all pixels categorized as liver are aggregated, and the aggregated region is represented by a label value corresponding to the liver. The label value here is set according to the specific coarse segmentation task. The segmentation mask refers to a corresponding mask obtained after a segmentation operation. In some embodiments, the mask may include the organ mask (e.g., an organ mask of the target organ) and the vascular mask.

In some embodiments, in the fast segmentation mode, only the thoracic cavity or abdominal cavity is taken as an example. Firstly, the region positioning calculation is performed on the thoracic cavity or abdominal cavity within the scanning range of the intraoperative scanning image. Specifically, for the abdominal cavity, the top of the liver to the bottom of the rectum is selected as a positioning region of the abdominal cavity. For the thoracic cavity, the top of the esophagus to the bottom of the lungs (or the top of the liver) as the positioning region of the thoracic cavity. After determining regional positioning information of the thoracic cavity or abdominal cavity, the thoracic cavity or abdominal cavity is segmented and further segmented within the segmentation region to extract an interventional region (as opposed to the non-interventional region, such as the penetrable region, fat, etc.). Finally, using an abdominal segmentation mask to remove a segmentation mask and a penetrable region mask of the target organ, i.e., the non-interventional region may be extracted. In some embodiments, the intervenable region may include a fatty portion, such as a gap between two organs that contains fat, etc. In the case of the liver, a portion of the region between the subcutaneous tissue to the liver may be covered with fat. Image processing efficiency is improved due to the fast processing speed in the fast segmentation mode, which in turn makes planning faster and shorter.

In some embodiments, in the fine segmentation mode, all organs of the scanned image during surgery can be segmented. In some embodiments, all of the organs of the intraoperative scanning image may include essential organs and vital organs of the intraoperative scanning image. In some embodiments, the essential organs of the intraoperative scanning image may include a target organ of the intraoperative scanning image. In some embodiments, predetermined vital organ(s) of the intraoperative scanning image may be segmented in the fine segmentation mode. The predetermined vital organs may be determined based on the level of importance of each organ of the intraoperative scanning image. For example, all of the vital organs in the intraoperative scanning image may be determined as the predetermined vital organs. In some embodiments, a ratio of the total volume of the predetermined vital organs in the fast segmentation mode to the total volume of the predetermined vital organs in the fine segmentation mode may be greater than a predetermined efficiency factor m. The predetermined efficiency factor m may be used to characterize a difference in segmentation efficiency (or the detail level of segmentation) for segmentation based on different segmentation modes. In some embodiments, the predetermined efficiency factor m may be equal to or greater than 1. In some embodiments, the setting of the efficiency factor m is related to a type of the interventional procedure. The type of the interventional procedure may include, but is not limited to, a urological procedure, a thoracic and abdominal procedure, a cardiovascular procedure, an obstetrics and gynecology intervention procedure, a skeletal muscle procedure, or the like. Merely by way of example, the predetermined efficiency factor m in the urological procedure may be set larger; the predetermined efficiency factor m in the thoracic and abdominal procedure may be set smaller.

In some embodiments, segmentation masks of all organs of the intraoperative scanning image are obtained by segmentation in the fine segmentation mode. In some embodiments, in the fine segmentation mode, the segmentation masks and vascular masks of all organs of the intraoperative scanning image are obtained by segmentation. In some embodiments, in the fine segmentation mode, the vascular masks within all organs are determined based on the segmentation masks and vascular masks of all organs of the intraoperative scanning image. As can be seen, the content of the segmented image is more detailed in the fine segmentation mode, making the planned path more selective and enhancing the robustness of image processing.

More description regarding each of the above operations may be found in operation 220 of FIG. 2, and will not be repeated here.

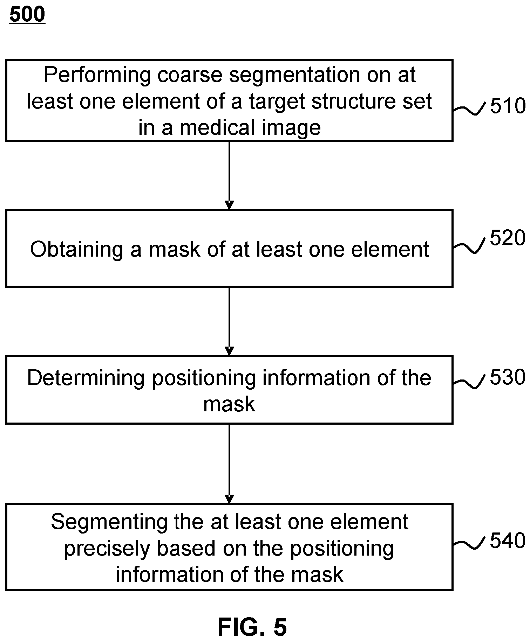

FIG. 5 is a flowchart illustrating an exemplary process of segmentation in medical image processing for an interventional procedure according to some embodiments of the present disclosure. As shown in FIG. 5, the process 500 may include the following operation.

In 510, coarse segmentation may be performed on at least one element of a target structure set in a medical image.

In some embodiments, the medical image may include a preoperative enhanced image and an intraoperative scanning image. The target structure set may include any one or more of a first target structure set, a second target structure set, and a fourth target structure set.

In some embodiments, in operation 510, the processing device may utilize a threshold segmentation algorithm, a region growing algorithm, or a level-set algorithm, to perform the coarse segmentation on the at least one element of the target structure set in the medical image. Elements may include the target organ in the medical image, blood vessels within the target organ, a lesion, a non-interventional region, all vital organs, etc. In some embodiments, the coarse segmentation based on the threshold segmentation algorithm may be implemented as follows. A plurality of different pixel threshold ranges may be set, based on pixel values of the inputted medical image, each pixel in the medical image may be categorized, and pixel points with pixel values within the same pixel threshold range are segmented into the same region. In some embodiments, the coarse segmentation based on the region growing algorithm may be implemented as follows. According to a known pixel point or a predetermined region including pixel points on the medical image, a similarity determination condition is predetermined based on a demand; according to the predetermined similarity determination condition, a pixel point is compared with its neighboring pixel points, or the predetermined region is compared with its neighboring regions, and pixel points or regions with high similarity are merged until the above process cannot be repeated, and then the merging terminates, and the coarse segmentation is completed. In some embodiments, the predetermined similarity determination condition may be determined based on a predetermined image feature, exemplarily, an image feature such as grayscale, texture, or the like. In some embodiments, the coarse segmentation based on a level-set algorithm may be implemented as follows. A target contour of the medical image is determined as a zero level set of a high-dimensional function, which is discretized, and the output is extracted from the zero level set to obtain a contour of a target, and then pixel region within the contour are segmented.

In some embodiments, the processing device may utilize a deep learning convolutional network-based algorithm to perform the coarse segmentation on at least one element of the target structure set in the medical image. In some embodiments, the deep learning convolutional network-based algorithm may include a segmentation algorithm based on a full convolutional network. In some embodiments, the convolutional network may utilize a network framework based on a U-shaped structure, such as UNet. In some embodiments, a network framework of the convolutional network may include an encoder, a decoder, and a skip connection structure. The encoder and the decoder may include a convolutional layer or a convolutional layer combined with an attention mechanism. The convolutional layer may be configured to extract features, and the attention mechanism may be configured to apply more attention to a focus region. The skip connection structure may be configured to combine features extracted by the encoder in different dimensions into the decoder, and finally output a segmentation result via the decoder. In some embodiments, the deep learning convolutional network-based algorithm for coarse segmentation may be implemented as follows. Feature extraction may be performed on the medical image by the encoder of the convolutional neural network through convolution, and then the decoder of the convolutional neural network may recover the extracted features into a pixel-level segmentation probability map. The segmentation probability map may represent a probability that each pixel point in the image belongs to a particular class, and finally the segmentation probability map may be output as a segmentation mask, thereby completing the coarse segmentation.

In 520, a mask of at least one element may be obtained.

A mask of an element (also referred to as element mask) refers to information used to mask an element in a target structure set. In some embodiments, the result of the coarse segmentation (e.g., the segmentation mask) may be used as the mask of the element.

In 530, positioning information of the mask may be determined.

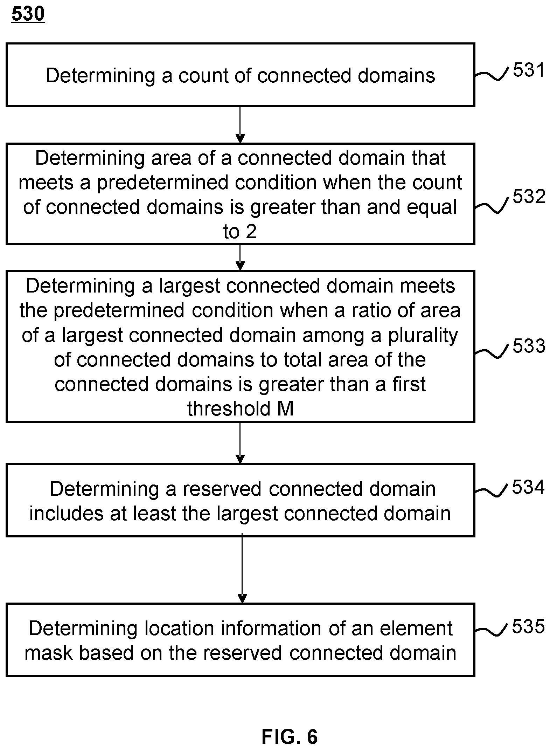

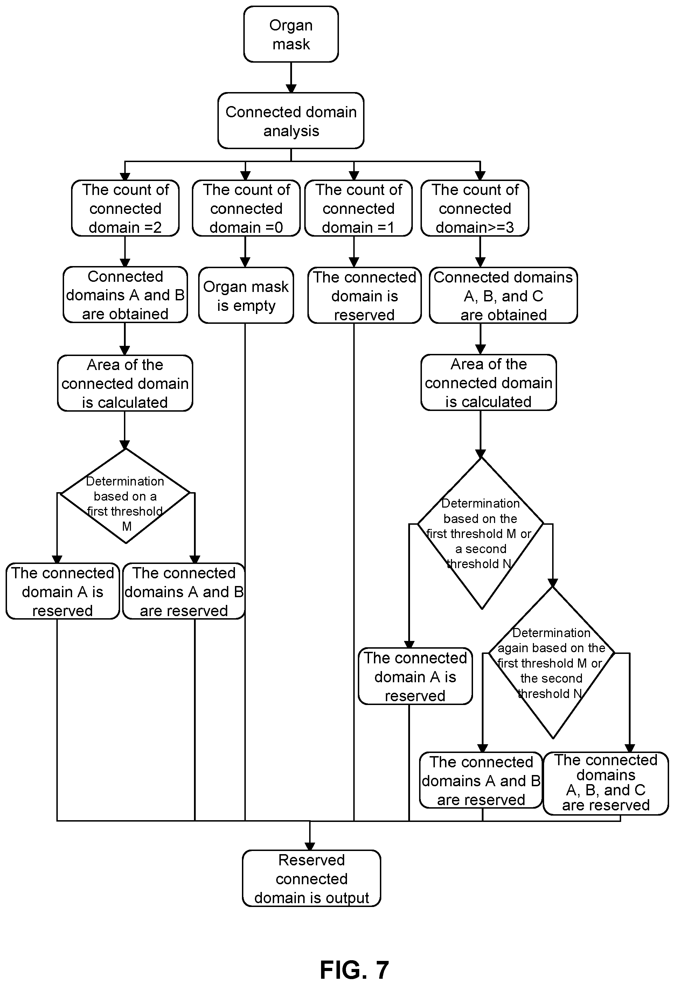

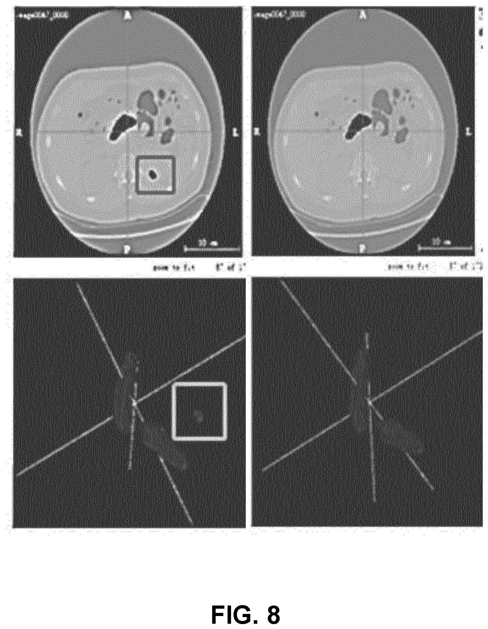

FIG. 6 is a flowchart illustrating an exemplary process for determining positioning information of an element mask according to some embodiments of the present disclosure. FIG. 7 is a flowchart illustrating an exemplary process of a soft connected component analysis of an element mask according to some embodiments of the present disclosure. FIG. 8 is a flowchart illustrating an exemplary process of a coarse segmentation for a soft connected component analysis of an element mask according to some embodiments of the present disclosure.

In some embodiments, in operation 530, the determining the positioning information of the mask may be implemented as follows. A soft connected component analysis may be performed on the element mask. The connected component, or a connectivity region, generally refers to an image region including foreground pixel points in the image that have the same pixel value and are adjacent to each other.

In some embodiments, operation 530, in which a soft connected component analysis of an element mask is performed, may include the following operations.

In 531, a count of the connected components may be determined.

In 532, in response to the count of the connected components being greater than or equal to 2, an area of the connected component(s) that meet a predetermined condition may be determined.

In 533, in response to a ratio of an area of a largest connected component among the plurality of connected components to a total area of the connected components being greater than a first threshold M, it may be determined that the largest connected component meets the predetermined condition.

In 534, it may be determined that a reserved connected component includes at least the largest connected component.

In 535, position information of the element mask may be determined based on the reserved connected component.

The predetermined condition refers to a condition that needs to be met for a connected component to function as a reserved connected component. For example, the predetermined condition may be a qualifying condition on the area of the connected component. In some embodiments, a plurality of connected components may be included in the medical image, and the plurality of connected components have different areas. The plurality of connected components having different areas may be sorted according to the sizes of the areas, e.g., from largest to smallest, and the sorted connected components may be notated as a first connected component, a second connected component, and a kth connected component. The first connected component may be a connected component with the largest area among the plurality of connected components, also referred to as the largest connected component. In this case, the predetermined condition for determining connected components with different area ordinal bits as the reserved connected components may be different, as described in FIG. 5. In some embodiments, connected components that meet the predetermined condition may include connected components whose areas are sorted in descending order of the area of the connected components within a predetermined ordinal bit n. For example, if the predetermined ordinal bit n is 3, each connected component may be judged whether the connected component is the reserved connected component according to the order of the area ordinal bit and according to the corresponding predetermined condition in turn. That is, first, it is determined whether the first connected component is the reserved connected component, and then, it is determined whether the second connected component is the reserved connected component. In some embodiments, the predetermined ordinal bit n may be set based on a type of the target structure, e.g., a thoracic target structure, or an abdominal target structure. In some embodiments, the first threshold M may take a value in a range of 0.8 to 0.95, the soft connected component analysis may be ensured to obtain a desired accuracy rate within the range of values. In some embodiments, the first threshold M may take a value in the range of 0.9 to 0.95, which further improves the accuracy of the soft connected component analysis. In some embodiments, the first threshold M may be set based on the type of the target structure, for example, the thoracic target structure, or the abdominal target structure. In some embodiments, the predetermined ordinal bit n or the first threshold M may also be reasonably set based on machine learning and/or big data and is not further limited herein.

In some embodiments, operation 530, in which the soft connected component analysis is performed on the element mask, may be performed as follows.

Based on the obtained elemental mask, the count of the connected component and corresponding areas within the element mask are analyzed and calculated as follows.

If the count of the connected component is 0, it means that the corresponding mask is empty, i.e., mask acquisition or coarse segmentation fails or the segmentation object does not exist, and no operation is performed. For example, when segmenting the spleen in the abdominal cavity, there may be a case of splenectomy, in which case the mask for the spleen is empty.

If the count of the connected component is 1, it means that there is only one connected component, and no false positives, no splitting and disconnecting, etc., and the connected component is reserved. It is understandable that if the count of the connected component is 0 and 1, there is no need to judge whether the connected component is reserved or not based on the predetermined condition.

If the count of the connected component is 2, the connected components A and B are obtained according to the size of the area (S), where the area of the connected component A is larger than the area of the connected component B, i.e., $S(A) > S(B)$. In conjunction with the above, the connected component A may be referred to as the first or largest connected component; the connected component B may be referred to as the second connected component. If the count of the connected component is 2, the predetermined condition that needs to be met for the connected component to be used as the reserved connected component may be a magnitude between a ratio of the area of the largest connected component to the total area of the connected component and a threshold. Calculations are performed on the connected component, if the proportion of the area of A to the total area of A and B is greater than the first threshold M, i.e., S(A)/S(A+B) is greater than the first threshold M, the connected component B may be determined as a false positive domain and only the connected component A is reserved (i.e., determining the connected component A as the reserved connected component). If the proportion of the area of A to the total area of A and B is less than or equal to the first threshold M, both A and B may be determined as a part of the element mask, while preserving the connected components A and B (i.e., determining the connected components A and B as the reserved connected components).

If the count of connected component is greater than or equal to 3, the connected components A, B, C...P are obtained according to the size of the area (S), where the area of the connected component A is greater than the area of the connected component B, the area of the connected component B is greater than the area of the connected component C, etc., i.e., S(A)>S(B)>S(C)>...>S(P). Then, the total area S(T) of the connected components A, B, C...P are calculated, at this time, whether each connected component (or the connected component whose area order is within the predetermined ordinal bit n) is the reserved connected component may be determined according to the order of the area ordinal bit and the corresponding predetermined condition in turn. In some embodiments, when the count of the connected component is greater than or equal to 3, the predetermined condition to be satisfied for the largest connected component (i.e., the connected component A) to be used as the reserved connected component may be the magnitude between the ratio of the area of the largest connected component to the total area of the connected component and the threshold (e.g., the first threshold M). In some embodiments, if the count of the connected components is greater than or equal to 3, the predetermined condition to be satisfied for the largest connected component (i.e., the connected component A) to be used as the reserved connected component may also be a magnitude between a ratio of the area of the second connected component to the area of the largest connected component and the threshold (e.g., the second threshold N). Specifically, if a proportion of the area of the connected component A to the total area S(T) is greater than the first threshold M, i.e., S(A)/S(T)>the first threshold M, or, if a proportion of the area of the connected component B to the area of the connected component A is less than the second threshold N, i.e., S(B)/S(A)<the second threshold N, the connected component A is determined to be an element mask and is reserved (i.e., the connected component A is the reserved connected component), and the remaining connected components are all determined to be false positive regions. Otherwise, the calculation continues, i.e., whether the second connected component (i.e., the connected component B) is the reserved connected component continues to be determined. In some embodiments, the predetermined condition to be satisfied by the connected component B as the reserved connected component may be a magnitude of a ratio of a sum of the areas of the first connected component and the second connected component to the total area of the connected component and the first threshold M. In some embodiments, the predetermined condition to be satisfied for the connected component B to be the reserved connected component may also be a magnitude of a proportion of the area of the third connected component to the sum of the areas of first connected component and the second connected component and the threshold (e.g., the second threshold N). Specifically, when a proportion of the sum of areas of the connected component A and the connected component B to the total area S(T) is greater than the first threshold M, i.e., S(A+B)/S(T)>the first threshold M, or, when the proportion of the area of the connected component C to the sum of areas of the connected component A and the connected component B is less than the second threshold N, i.e., S(C)/S(A+B)<the second threshold N, the connected components A and B are determined to be element masks and reserved (i.e. the connected component A and the connected component B are the reserved connected components), and the remaining parts are determined to be false positive regions. Otherwise, the calculation continues, i.e., whether the third connected component (i.e., the connected component C) is the reserved connected component continues to be determined. The manner of determining the connected component C is similar to the manner of determining the connected component B. The predetermined condition to be satisfied for the connected component C to be the reserved connected component may be a magnitude between a ratio of a sum of the areas of the first connected component, the second connected component, and the third connected component to the total area of the connected component and the first threshold M. Alternatively, a magnitude between a percentage of the area of the fourth connected component to a sum of the area of the first connected component, the area of the second connected component, and the area of the third connected component and the threshold (e.g., the second threshold N). Specifically, when the proportion of the sum of the areas of the connected component A, the connected component B, and the connected component C to the total area S(T) are greater than the first threshold M, i.e., S(A+B+C)/S(T)>the first threshold M, or, when the proportion of the area of the connected component D to the sum of the areas of the connected component A, the connected component B, and the connected component C is less than the second threshold N, i.e., S(D)/S(A+B+C)<the second threshold N, the connected components A, B and C are all determined as element masks and reserved (i.e., the connected component A, the connected component B, and the connected component C are all reserved connected components). With reference to the above determination manner, it can be sequentially determined whether the connected components A, B, C, D...P, or a portion of the connected components with an area ordinal bit within the predetermined ordinal bit n, are reserved connected components. It should be noted that only a judgment of whether three connected components are reserved connected components is shown in FIG. 6. It can also be understood that the value of the predetermined ordinal bit n in FIG. 6 is set to 4, and thus it only needs to determine whether the connected components with ordinal bits 1, 2, and 3, i.e., the connected component A, the connected component B, and the connected component C, are the reserved connected components.

Finally, the reserved connected components are output.

In some embodiments, the second threshold N may take a value in a range of 0.05 to 0.2, and within the range, the soft connected component analysis may be ensured to obtain a desired accuracy rate. In some embodiments, the second threshold N may be 0.05, which may achieve excellent accuracy in soft connected component analysis.

As shown in FIG. 8, the top and bottom of the left side show a cross-sectional medical image and a stereo medical image of a coarse segmentation result, respectively, without using soft connected component analysis, and the top and bottom of the right side show a cross-sectional and a stereo medical image of the coarse segmentation result, respectively, using the soft connected component analysis. As can be seen from the comparison, the results of the coarse segmentation performed on the element mask based on the soft connected component analysis show that the false positive region indicated by the box in the image on the left is removed, which is more accurate and more reliable than the previous connected component analysis manner in excluding the false positive region. Further, it directly helps the subsequent reasonable extraction of the bounding rectangular box of the positioning information of the element mask, which improves the segmentation efficiency.

In some embodiments, the positioning information of the element mask may be information about the position of a bounding rectangle of the element mask, such as coordinates information of a bounding rectangular box line of the bounding rectangle. In some embodiments, the bounding rectangle of the element mask covers a positioning region of the element. In some embodiments, the bounding rectangle may be displayed in the medical image in the form of a bounding rectangular box. In some embodiments, the bounding rectangle may be a bounding rectangular box constructed relative to the element mask based on bottom edges of various directions of the connected component of the element, e.g., the bottom edges in the top, bottom, left, and right directions of the connected component.

In some embodiments, the bounding rectangle of the element mask may be a rectangular box or a combination of rectangular boxes. For example, it may be a rectangular box of a larger area or a combination of a plurality of rectangular boxes of a smaller area pieced together to form a rectangular box of a larger area.

In some embodiments, the bounding rectangle of the element mask may be a bounding rectangular box where only one rectangular box exists. For example, if there is only one connected component in an element (e.g., a blood vessel or an organ in the abdominal cavity), a bounding rectangle of larger area may be constructed based on the bottom edge of each direction of that connected component. In some embodiments, the large bounding rectangle described above may be applied to an organ in which there is a connectivity domain.

In some embodiments, the bounding rectangle of the element mask may be a bounding rectangular box formed by a combination of a plurality of rectangular boxes. For example, if an element has a plurality of connected components, the plurality of connected components corresponding to a plurality of rectangular boxes, and a rectangular box is constructed based on bottom edges of the plurality of rectangular boxes. It is to be understood that, if bottom edges of three rectangular boxes corresponding to three connected components form a total bounding rectangular box, the calculation is performed according to the one total bounding rectangular box, which guarantees the realization of expected accuracy while reducing the calculation amount.

In some embodiments, if the medical image includes a plurality of connected components, the positioning information of the plurality of connected components may be first judged, and then the positioning information of the element mask may be obtained based on the positioning information of the plurality of connected components. For example, the connected component among the plurality of connected components that meet a predetermined condition, i.e., the positioning information of the reserved connected component, may be judged first, and then the positioning information of the element mask is obtained based on the positioning information of the reserved connected component.

In some embodiments, in operation 530, in which the positioning information of the element mask is determined may further include the operation of positioning the element mask based on the predetermined positioning coordinates of the element.

In some embodiments, this operation may be performed in the event that the positioning of the bounding rectangle of the element mask fails. It will be appreciated that when the coordinates of the bounding rectangle of the element mask do not exist, it is determined that the corresponding element has failed to be positioned.

In some embodiments, the predetermined element may be selected as an element that is more stable in positioning (e.g., an organ that is more stable in positioning), with a lower probability of positioning failure when positioning the element, thereby enabling precise positioning of the element mask. In some embodiments, the liver, stomach, spleen, and kidney are more stable due to the lower probability of failure to localize the liver, stomach, spleen, and kidneys in the abdominal cavity, and the lower probability of failure to localize the lungs in the thoracic cavity, and the more stable positioning of these organs, kidney may be used as predetermined organs in the abdominal cavity, i.e., the predetermined elements may include the liver, the stomach, the spleen, the kidneys, the lungs, or any other possible organs and tissues. In some embodiments, organ masks in the abdominal cavity may be positioned again based on the positioning coordinates of the liver, the stomach, the spleen, and the kidney. In some embodiments, the organ masks in the thoracic cavity may be positioned based on the positioning coordinates of the lungs.

In some embodiments, the element mask may be positioned again using the predetermined positioning coordinates of the element as the reference coordinates. In some embodiments, if the element failed to be positioned is in the abdominal cavity, then the positioning coordinates of the liver, the stomach, the spleen, and the kidneys may be used as the coordinates for re-positioning, whereby the element failed to be positioned in the abdominal cavity is re-positioned. In some embodiments, if the element failed to be positioned is in the thoracic cavity, then the positioning coordinates of the lungs may be used as the positioning coordinates for re-positioning, whereby the element failed to be positioned in the thoracic cavity is re-positioned. Merely by way of example, if the element whose positioning has failed is in the abdominal cavity, the positioning coordinates of the top of the liver, the bottom of the kidney, the left side of the spleen and the right side of the liver may be used as the coordinates of the cross-sectional defense line (upper side, lower side) and the coronal direction (left side, right side) for the re-positioning, and the most front and the most rear end of the coordinates of the four organs may be taken as the coordinates of the sagittal direction (front and rear) for the new positioning, according to which the element whose positioning has failed in the abdominal cavity may be re-positioned. Merely by way of example, if the failed positioning element is in the thoracic cavity, the bounding box formed by the positioning coordinates of the lungs may be expanded by a certain count of pixels, whereby the failed positioning element in the chest is re-positioned.

Precise positioning of the element mask based on the predetermined positioning coordinates of the element may improve the segmentation accuracy, with the reduction of the segmentation time, which improves the efficiency of the segmentation, and at the same time reduces the amount of segmentation calculation and saves memory resources.

In 540, fine segmentation may be performed on the at least one element based on the positioning information of the mask.

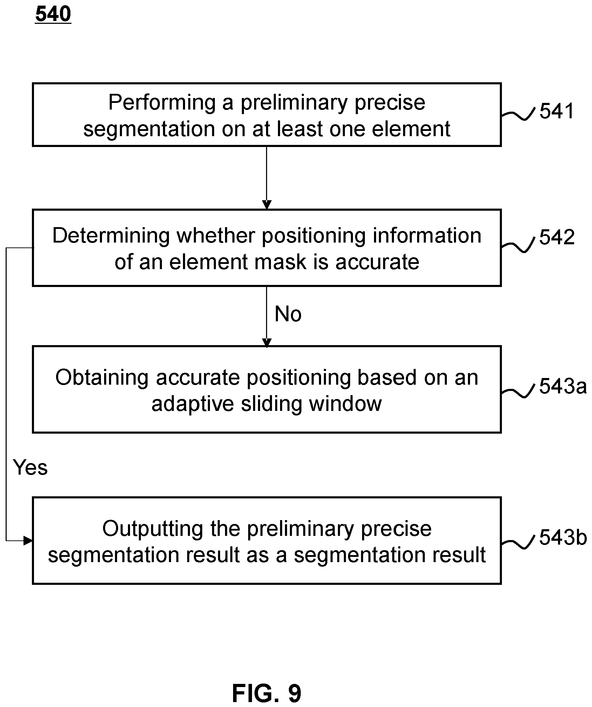

FIG. 9 is a flowchart illustrating an exemplary process of a fine segmentation of an element according to some embodiments of the present disclosure.

In some embodiments, in operation 540, in which the fine segmentation is performed on the at least one element based on the positioning information of the mask may include the following operations.

In 541, a preliminary fine segmentation may be performed on at least one element. The preliminary fine segmentation may be a fine segmentation based on positioning information of a coarsely segmented element mask. In some embodiments, the preliminary fine segmentation of the element may be performed based on input data and a bounding box positioned by the coarse segmentation. A precisely segmented element mask may be generated by the preliminary fine segmentation.

In 542, whether the positioning information of the element mask is accurate may be determined. By operation 542, it may be judged whether the positioning information of the element mask obtained by coarse segmentation is accurate or not, and it may be further judged whether the coarse segmentation is accurate or not.

In some embodiments, the element mask of the preliminary fine segmentation may be calculated to obtain the positioning information thereof, and the positioning information of the coarse segmentation may be compared with the positioning information of the fine segmentation. In some embodiments, a bounding box of the coarsely segmented element mask may be compared with a bounding box of the precisely segmented element mask to determine a magnitude of a difference between the two. In some embodiments, the bounding box of the coarsely segmented element mask may be compared, in six directions in three-dimensional space (i.e., the entirety of the bounding box is a cube in three-dimensional space), to the bounding box of the precisely segmented element mask to determine the magnitude of the difference. Merely by way of example, an overlapping degree between each edge of the bounding box of the coarsely segmented element mask and each edge of the bounding box of the precisely segmented element mask may be calculated, or a difference between coordinates of the 6 vertices of the bounding box of the coarsely segmented element mask and the bounding rectangular box of the precisely segmented element mask may be calculated.

In some embodiments, the positioning information of the coarsely segmented element mask may be determined based on the positioning information of the preliminary precisely segmented element mask. In some embodiments, whether the judgment result is precise may be determined based on the magnitude of the difference between the positioning information of the coarse segmentation and the positioning information of the fine segmentation. In some embodiments, the positioning information may be a bounding rectangle (e.g., a bounding box) of the element mask, and based on the bounding rectangle of the coarsely segmented element mask and the bounding rectangle of the accurately segmented element mask, it is determined whether the bounding rectangle of the coarsely segmented element mask is accurate. At this time, the magnitude of the difference between the positioning information of the coarse segmentation and the positioning information of the fine segmentation may be the magnitude of a distance between the closest bounding rectangular box lines in the bounding box of the coarse segmentation and the bounding box of the fine segmentation. In some embodiments, if the difference between the positioning information of the coarse segmentation and the positioning information of the fine segmentation is large (i.e., the distance between the closest bounding rectangular box lines in the bounding rectangular box of the coarse segmentation and the bounding rectangular box of the fine segmentation is large), then the positioning information of the coarse segmentation may be judged to be accurate. If the difference is small (i.e., the distance between the closest bounding rectangular box lines in the bounding rectangular box of the coarse segmentation and the bounding rectangular box of the fine segmentation is small), then the positioning information of the coarse segmentation may be judged to be inaccurate. It should be noted that the bounding rectangular box of the coarse segmentation is obtained by expanding pixels (e.g., by 15-20 voxels) on a bounding rectangular box line of an original coarse segmentation, and the bounding rectangular box line is close to the element. In some embodiments, it may be possible to determine, based on a magnitude relationship between the distance between the closest bounding rectangular box lines in the bounding rectangular box of the coarse segmentation and the bounding rectangular box of the fine segmentation and a predetermined threshold, whether the positioning information of the coarse segmentation is accurate. For example, the distance may be determined to be inaccurate when the distance is less than the predetermined threshold, and the distance may be determined to be accurate when the distance is greater than the predetermined threshold. In some embodiments, in order to ensure the judgment accuracy, the predetermined threshold may be less than or equal to 5 voxels.

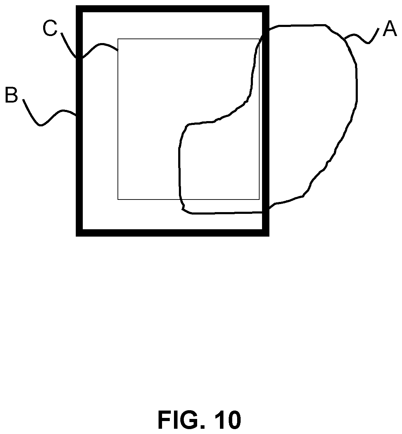

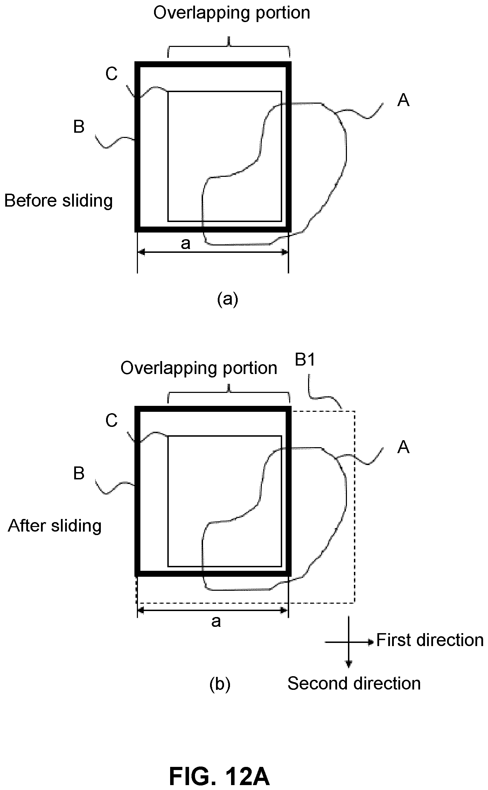

FIG. 10 to FIG. 11 are schematic diagrams illustrating determining positioning information of an element mask according to some embodiments of the present disclosure. FIG. 12A is a schematic diagram illustrating determining a sliding direction based on positioning information of an element mask according to some embodiments of the present disclosure.

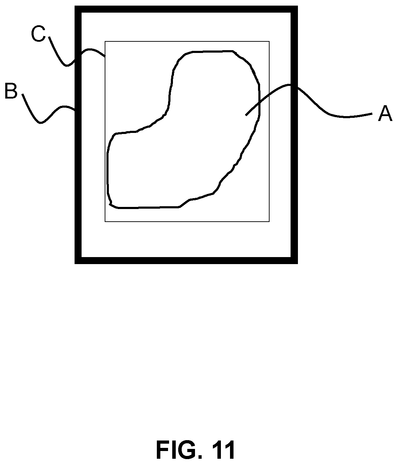

FIGs. 10 and 11 show the element mask A obtained by coarse segmentation, a bounding rectangular box B of the element mask A (i.e., positioning information of the element mask A), and a bounding rectangular box C after the preliminary fine segmentation based on the bounding rectangular box of the coarse segmentation. FIG. 12A shows a sliding window B1 obtained after the sliding window of the bounding rectangular box B of the coarse segmentation. Diagram (a) in FIG. 12A is a schematic diagram before sliding window, and diagram (b) is a schematic diagram after sliding window. Alternatively, for convenience, to illustrate by way of example with a plane rectangular box in one plane of a three-dimensional bounding rectangular box, it may be understood that there exist other 5 plane rectangular boxes in the three-dimensional bounding rectangular box, i.e., in carrying out the specific calculations of the three-dimensional bounding rectangular box, there exists 6 directions of the bounding box line, here only 4 bounding box lines in a certain plane are illustrated.

Merely by way of example, as shown in FIG. 10, the right bounding box line in the bounding rectangular box C of the fine segmentation is less different from the corresponding bounding box line in the bounding rectangular box B of the coarse segmentation. From this, it can be judged that the bounding rectangular box B of the coarse segmentation is inaccurate in a direction corresponding to the right bounding box line, and the right bounding box line needs to be adjusted. However, the upper, lower, and left bounding box lines in the bounding rectangular box C differ significantly from the upper, lower, and left bounding box lines in the bounding rectangular box B. Thus, it can be determined that the bounding rectangular box B of the coarse segmentation is accurate in the direction corresponding to the upper, lower, and left bounding box lines. Merely by way of example, as shown in FIG. 11, a difference between the bounding box lines of 4 edges in the bounding rectangular box C of the fine segmentation and the corresponding bounding box lines in the bounding rectangular box B of the coarse segmentation is large. Thus, it can be judged that the bounding box lines of the 4 edges in the bounding rectangular box B of the coarse segmentation are accurate. It should be noted that there are 6 directions in the element mask A, and FIG. 10 and FIG. 11 only illustrate 4 bounding box lines. In practice, it may be judged on 12 bounding box lines in 6 directions in the element mask A.

In 543a, if the result of the judgment is inaccurate, accurate positioning information may be obtained based on an adaptive sliding window. In some embodiments, if the result of coarse segmentation is inaccurate, elements obtained by fine segmentation may be more likely inaccurate, and the corresponding adaptive sliding window calculation may be performed thereon, and the accurate positioning information may be obtained in order to continue fine segmentation.

In some embodiments, obtaining the accurate positioning information based on the adaptive sliding window may be implemented as follows. At least one direction in which the positioning information is inaccurate is determined. The adaptive sliding window calculation is performed in the direction based on an overlap rate parameter. In some embodiments, the at least one direction in which the bounding rectangular box is inaccurate may be determined. After determining that the bounding rectangular box of the coarse segmentation is inaccurate based on a preset overlap rate parameter, the bounding rectangular box of the coarse segmentation may slide in the corresponding direction, i.e., a sliding window operation is performed, and the sliding window operation is repeated until all the bounding rectangular boxes are completely accurate. The overlap rate parameter refers to a ratio of an area of an overlapped portion of an initial bounding rectangular box with the initial bounding rectangular box after sliding window to an area of the initial bounding rectangular box. If the overlap rate parameter is higher, a sliding step size of the sliding window operation may be shorter. In some embodiments, the overlap rate parameter may be set smaller if it is desired to ensure that the process of sliding window calculation is more concise (i.e., there are fewer steps in the sliding window operation), and the overlap rate parameter may be set larger if it is desired to ensure that the results of the sliding window calculation are more accurate. In some embodiments, a sliding step size for performing the sliding window operation may be calculated based on a current overlap rate parameter. According to the judgment manner of FIG. 10, it can be seen that the bounding rectangular box B of the coarse segmentation in FIG. 12A is inaccurate in the direction corresponding to the right and lower bounding box lines. For ease of description, the direction corresponding to the right bounding box line of the bounding rectangular box B is here notated as a first direction (the first direction is perpendicular to the right bounding box line of the bounding rectangular box B), and the direction corresponding to the lower bounding box line is notated as a second direction (the second direction is perpendicular to the lower bounding box line of the bounding rectangular box B). Merely by way of example, as shown in FIG. 12A, assuming that a length of the bounding rectangular box B is a, when the overlap rate parameter is 60%, it can be determined that the corresponding step size is a*(1-60%). As described above, the right bounding box line of the bounding rectangular box B may slide along the first direction by a*(1-60%). Similarly, the lower bounding box line of the bounding rectangular box B may slide along the second direction by the corresponding step. The corresponding sliding window operation is repeated on the right bounding box line as well as the lower bounding box line of the bounding rectangular box B, respectively, until the bounding rectangular box B is completely accurate, as shown in the sliding window B1 in (b) in FIG. 12A. Combined with FIGs. 10 and 12A, when it is determined that the bounding rectangular box of the coarse segmentation (i.e., the positioning information of the target structure mask) is inaccurate, coordinate values of the bounding box lines in 6 directions on the bounding rectangular box of the fine segmentation are compared one by one with coordinate values of the bounding box lines in 6 directions on the bounding rectangular box of the coarse segmentation. When a difference value is less than a coordinate difference threshold (e.g., the coordinate difference threshold is 5pt), it can be judged that the bounding box line of the bounding rectangular box is inaccurate in the direction. The coordinate difference value may be set according to the actual situation and will not be limited here.

Furthermore, for example, as shown in FIG. 10, pixel point coordinates of four directions corresponding to the four edges in an image of the bounding rectangular box C of the fine segmentation are compared one by one with pixel point coordinates of the four directions corresponding to four bounding box lines in the bounding rectangular box B of the coarse segmentation. When a difference between pixel point coordinates of one direction is less than the coordinate difference threshold of 8pt, it may be determined that the bounding rectangular box of the coarse segmentation in FIG.10 is inaccurate in the direction. For example, if the difference value of the upper edge is 20pt, the difference value of the lower edge is 30pt, the difference value of the right edge is 1pt, and the left edge is 50pt, then the direction corresponding to the right edge is inaccurate, and the direction corresponding to the upper edge, the lower edge, and the left edge is accurate.

As another example, in conjunction with FIG. 12A, B1 is a bounding rectangular box (also referred to as a sliding window) obtained by sliding the bounding rectangular box B of the coarse segmentation. It is understood that the sliding window is a bounding rectangular box of the coarse segmentation that meets a desired accuracy standard, and it is necessary to slide the bounding box lines of the bounding rectangular box of the coarse segmentation (e.g., a right bounding box line and a lower bounding box line) in corresponding directions (e.g., the first direction and the second direction) respectively in a corresponding step size to the position of the sliding window B1. The direction corresponding to each bounding box line that does not conform to the standard is moved in turn. For example, the right bounding box line of B may slide first, and then the lower bounding box line of B may slide to the specified position of the sliding window, whereas the direction corresponding to the left edge and the upper edge of B are standard, thus no need for sliding window. It is understood that the step size of each edge of the slide depends on the overlap rate of B1 and B, where the overlap rate may be a ratio of a current overlap area of the bounding rectangular box B of the coarse segmentation and the sliding window B1 to the total area. For example, the current overlap rate is 40%, etc. It should be noted that the bounding box lines of the bounding rectangular box B of the coarse segmentation may be slid in a left-to-right order, a top-to-bottom order, or any other feasible order, and is not further limited herein.

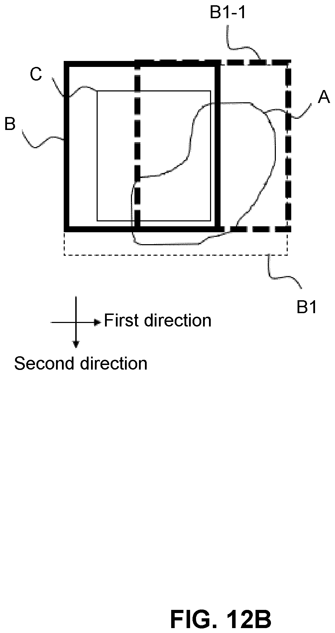

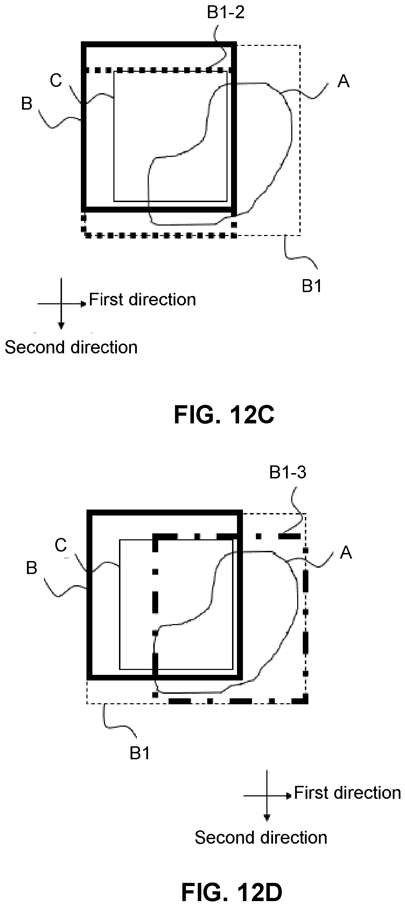

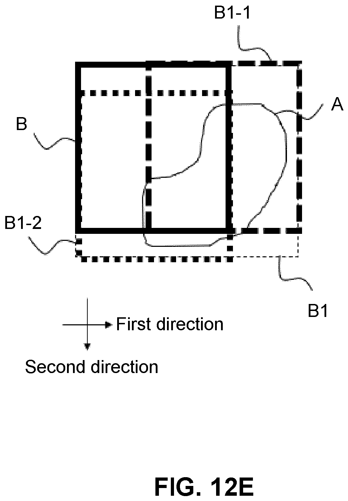

FIGs. 12B-12E are schematic diagrams illustrating a fine segmentation after a sliding window operation according to some embodiments of the present disclosure. Combined with FIGs. 12B-12E, in some embodiments, based on a bounding rectangular box of an original coarse segmentation (original sliding window), an accurate bounding rectangular box of the coarse segmentation is obtained after an adaptive sliding window operation, a coordinate value of the accurate bounding rectangular box may be obtained. Based on the coordinate value and the overlap rate parameter, a new sliding window is precisely segmented, and a fine segmentation result is superimposed on a preliminary fine segmentation result, and a final fine segmentation result is obtained. Specifically, referring to FIG. 12B, the sliding window operation may be performed on the original sliding window B to obtain a sliding window B1 (a bounding rectangular box of the largest range after the sliding window operation), the original sliding window B may slide along the first direction by a corresponding step size to obtain a sliding window B1-1, and then a fine segmentation is performed on the full domain range of the sliding window B1-1 to obtain a fine segmentation result of the sliding window B1-1. Further, referring to FIG. 12C, the original sliding window B may slide by the corresponding step size along the second direction to obtain the sliding window B1-2, and then the fine segmentation may be performed on the full domain range of the sliding window B1-2 to obtain a fine segmentation result. Further, referring to FIG. 12D, the original sliding window B may slide to obtain a sliding window B1-3 (e.g., the sliding window B1-2 may be obtained by performing the sliding window operation shown in FIG. 12C on the original sliding window, and then obtain the sliding window B1-3 by sliding the sliding window B1-2), and then the fine segmentation may be performed on the full domain range of the sliding window B1-3 to obtain a fine segmentation result of the sliding window B1-3. The fine segmentation results of the sliding window B1-1, the sliding window B1-2, and the sliding window B1-3 are superimposed on the preliminary fine segmentation result to obtain the final fine segmentation result. It should be noted that dimensions of the sliding window B1-1, the sliding window B1-2, and the sliding window B1-3 are the same as those of the original sliding window B. The sliding window B1 is a final sliding window result obtained by performing successive sliding window operations, i.e., the sliding window B1-1, the sliding window B1-2, and the sliding window B1-3, on the original sliding window B. In some embodiments, there may be a repetitive overlapping portion of the fine segmentation results of the sliding window B1-1, the sliding window B1-2, and the sliding window B1-3 when they are superimposed on the preliminary fine segmentation results. For example, in FIG. 12E, there may be an intersection portion between the sliding window B1-1 and the sliding window B1-2, and the intersection portion may be duplicated and superimposed when the segmentation results are superimposed. This situation may be handled in the following way. For a certain part of the element mask A, if the segmentation result of one sliding window is accurate for that part and the segmentation result of the other sliding window is inaccurate, the segmentation result of the sliding window with an accurate segmentation result may be taken as the segmentation result of the part. If the segmentation results of two sliding windows are accurate, the segmentation result of the right sliding window may be taken as the segmentation result of the part. If the segmentation results of two sliding windows are inaccurate, the segmentation result of the right sliding window may be taken as the segmentation result of the part, and the fine segmentation continues to be performed until the segmentation result is accurate.

In some embodiments, as shown in FIG. 9, when the judgment result is inaccurate, the operation of obtaining accurate positioning information based on the adaptive sliding window may be performed in a cyclic process. Specifically, after comparing the precisely segmented bounding box line and the coarsely segmented bounding box line, an updated coordinate value of the bounding rectangle box of the fine segmentation may be obtained by the adaptive sliding window. The bounding rectangle box of the fine segmentation is expanded by a certain number of pixels and then set as the bounding rectangle box of the coarse segmentation in a new cycle. Then the new bounding rectangle box is precisely segmented again to get a new bounding rectangle box of the fine segmentation, and whether it is accurate is calculated. If it is accurate, then the cycle may be terminated, otherwise the cycle may be continued. In some embodiments, a deep convolutional neural network model may be utilized to accurately segment at least one element in the coarse segmentation. In some embodiments, a historical medical image initially obtained before the coarse segmentation may be utilized as training data, and the deep convolutional neural network model may be obtained by training historical fine segmentation result data. In some embodiments, a historical medical image and a historical accurate segmentation result data are obtained from a historical scanned medical image of a scanning object and the historical fine segmentation result data by a medical scanning device. In some embodiments, the historical scanned medical image of the scanning object and the historical fine segmentation result data may be obtained from the terminal 130, the processing device 140, and the storage device 150.

In 543b, if the judgment result is accurate, the preliminary fine segmentation result may be output as the segmentation result.

In some embodiments, if the judgment result (i.e., the coarse segmentation result) is accurate, it can be determined that the positioning information of the element obtained by fine segmentation through the coarse segmentation result is accurate, and the preliminary fine segmentation result may be output.

In some embodiments, at least one element result data of the above performed fine segmentation may be output. In some embodiments, an image post-processing operation may be performed before the output of the segmentation result to further reduce noise and optimize the image display effect. The image post-processing operation may include performing edge smoothing on the image and/or image denoising, etc. In some embodiments, the edge smoothing may include smoothing or blurring to reduce noise or distortion in the medical image. In some embodiments, the smoothing or blurring process may be performed in various ways such as mean filtering, median filtering, Gaussian filtering, and bilateral filtering.

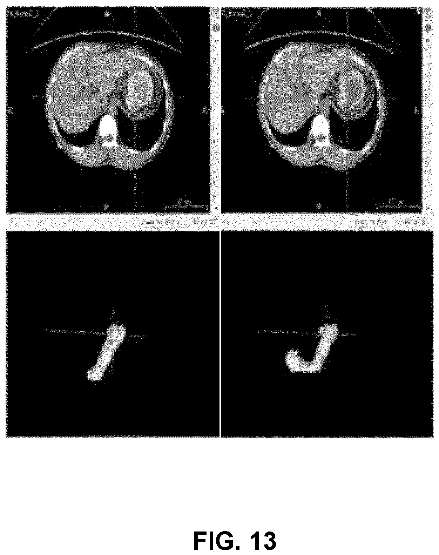

FIG. 13 is a diagram illustrating comparison of exemplary segmentation results according to some embodiments of the present disclosure.

As shown in FIG. 13, the top and bottom on the left side show a cross-sectional medical image and a stereo medical image, respectively, of the coarse segmentation result without using soft connected component analysis, and the right side show a cross-sectional and a stereo medical image, respectively, of the coarse segmentation result using the soft connected component analysis. After comparison, it can be seen that the obtained target organ in the segmentation result of the target organ shown in the segmentation result image on the right is more complete than that in the segmentation result of the target organ shown in the segmentation result image on the left, which reduces the risk of missing segmented organ and improves the segmentation accuracy rate.

In 460, the first medical image and the second medical image may be registered, and a spatial position of a third target structure set may be determined in the procedure.

More descriptions regarding determining the spatial position of the third target structure set in the procedure by registering may be found in the descriptions of operation 420.

In some embodiments, a fourth target structure set may also be considered to be a part of the third target structure set, e.g., a non-interventional region, all vital organs outside the target organ.

In some embodiments, the first medical image (i.e., a segmentation image of a preoperative first target structure set obtained by segmentation of a preoperative enhanced image) may include precise structural features of the first target structure set (e.g., blood vessels within a preoperative target organ, a preoperative target organ, and a preoperative lesion). The second medical image (i.e., a segmented image of an intraoperative second target structure set obtained by segmentation of an intraoperative scanning image) may include precise structural features of the second target structure set (e.g., an intraoperative target organ, an intraoperative lesion, an intraoperative non-interventional region or all vital organs). In some embodiments, the first medical image, the second medical image may be processed to separate appearance features of the target structure set from the background before registration. In some embodiments, the separation process of the appearance features from the background may be performed using an artificial neural network (a linear decision function, etc.), a threshold-based segmentation algorithm, an edge-based segmentation algorithm, an image segmentation algorithm based on cluster analysis (e.g., K-means, etc.), or any other feasible algorithms, such as wavelet transform-based segmentation algorithm, or the like.

The following is an exemplary description of the registration process using the first medical image including the blood vessels and the structural features of the preoperative target organ (i.e., the first target structure set includes the blood vessels within the target organ and the target organ) within the preoperative target organ, and the second medical image including the structural features of the intraoperative target organ, the intraoperative lesion, and intraoperative non-interventional region or all vital organs (i.e., the second target structure set includes the target organ, the lesion, the non-interventional region or all vital organs) as an example. It will be appreciated that the structural features of the lesion are not limited to being included in the second medical image, and in other embodiments, the structural features of the lesion may also be included in the first medical image, or the structural features of the lesion may be included in both the first medical image and the second medical image.

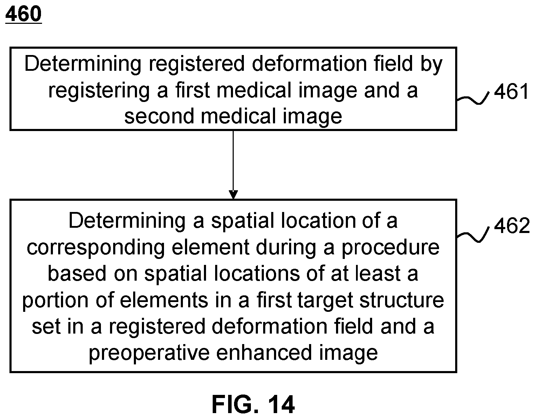

FIG. 14 is a flowchart illustrating an exemplary process for registering a first medical image and a second medical image according to some embodiments of the present disclosure.

In 461, the first medical image may be registered with the second medical image to determine a registration deformation field.

Descriptions regarding the registration and the registration process may be found in FIG.2 and will not be repeated here.

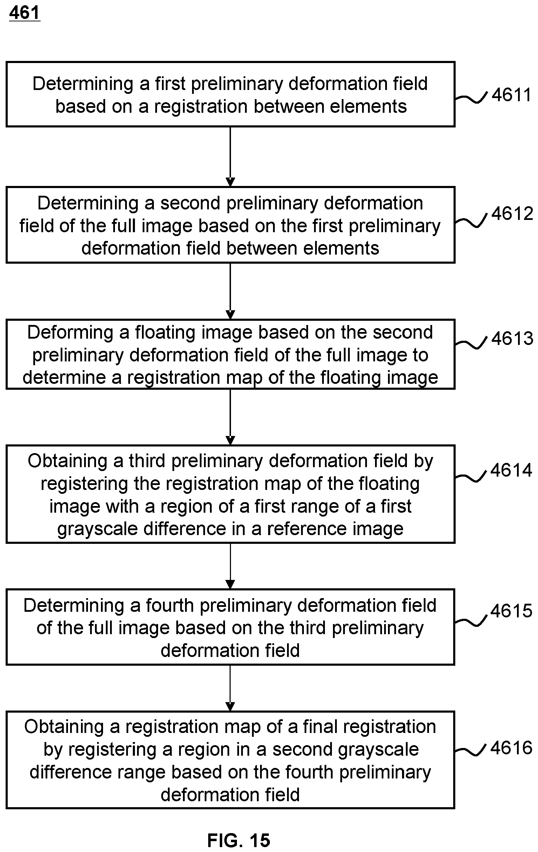

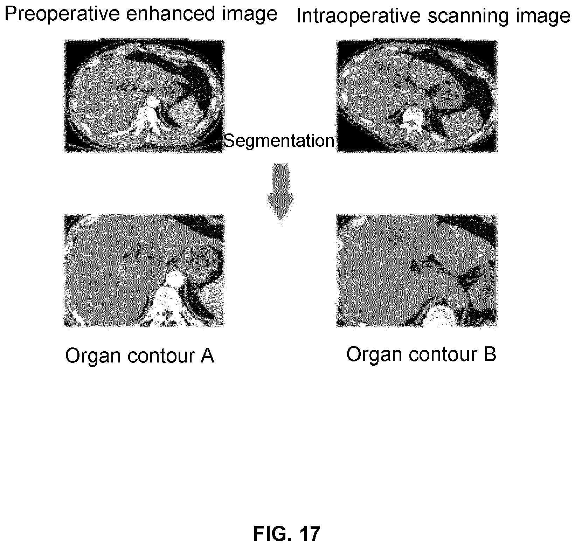

FIGs. 15 to 16 are flowcharts illustrating an exemplary process for determining a registration deformation field according to some embodiments of the present disclosure. FIG. 17 is a schematic diagram illustrating obtaining a first medical image and a second medical image by segmentation according to some embodiments of the present disclosure.

In some embodiments, the process of registering the first segmentation image with the second segmentation image to determine the registration deformation field, in operation 461, may include the following operations.

In 4611, a first preliminary deformation field may be determined based on a registration between elements.

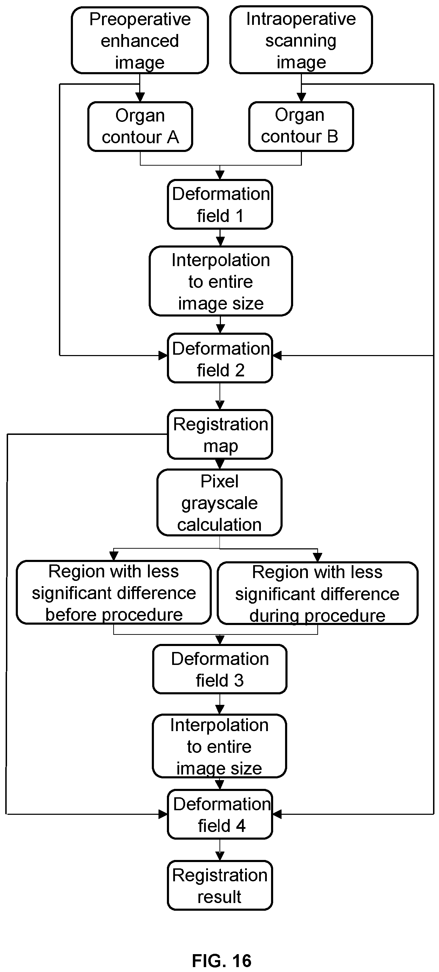

In some embodiments, the elements may be element contours (e.g., organ contours, vessel contours, lesion contours) of the first medical image and the second medical image. The registration between elements may refer to the registration between image regions covered by the element contours (masks). For example, in FIGs. 16 and 17, the preoperative enhanced image is segmented to obtain the image region (the region with the same or essentially the same grayscale within the dashed line region in the lower-left drawing) covered by the organ contour A of the target organ, and the intraoperative scanning image is segmented to obtain the image region (the region with the same or essentially the same grayscale within the dashed line region in the lower-right drawing) covered by the organ contour B of the target organ.

In some embodiments, the first preliminary deformation field (e.g., deformation field 1 in FIG. 16) is obtained by regional registration between the image region covered by the organ contour A and the image region covered by the organ contour B. In some embodiments, the first preliminary deformation field may be a local deformation field. For example, a local deformation field about the liver contour is obtained by the preoperative contour A of the liver with the intraoperative contour B.

In 4612, a second preliminary deformation field of the full image may be determined based on the first preliminary deformation field between elements.

The full image may be a regional image of the contained element. For example, if the target organ is the liver, the full image may be an image of the entire abdominal cavity. As another example, if the target organ is a lung, the full image may be an image of the entire thoracic cavity.

In some embodiments, the second preliminary deformation field of the full image may be determined by interpolation based on the first preliminary deformation field. In some embodiments, the second preliminary deformation field may be a global deformation field. For example, a deformation field 2 of the entire image size is determined by interpolation from deformation field 1 as shown in FIG. 16.

In 4613, a floating image is deformed based on the second preliminary deformation field of the full image to determine a registration map of the floating image.

The floating image may be an image to be registered, e.g., a preoperative enhanced image, an intraoperative scanning image. For example, when registering the intraoperative scanning image to a preoperative scanning image, the floating image is the intraoperative scanning image. The intraoperative scanning image may be registered to the preoperative scanning image spatially based on the registration deformation field. As another example, when registering the preoperative enhanced image to the intraoperative scanning image, the floating image is the preoperative enhanced image. The preoperative scanning image may be registered to the intraoperative scanning image spatially based on the registration deformation field. The registration map of the floating image may be an image of an intermediate registration result obtained during the registration process. Taking the example of registering the preoperative enhanced image to the intraoperative scanning image, the registration map of the floating image may be an intermediate intraoperative scanning image obtained during the registration process. For ease of understanding, embodiments of the present disclosure illustrate the registration process in detail by taking the example of registering the preoperative enhanced image to the intraoperative scanning image.

In some embodiments, as shown in FIG. 16, based on the deformation field 2 of the obtained full image, the floating image, i.e., the preoperative enhanced image, is deformed, and the preoperative enhanced image is determined to be a registration map of the preoperative enhanced image, i.e., the intraoperative scanning image of the intermediate registration result. For example, as shown in FIG. 16, the preoperative enhanced image (abdominal enhanced image) is deformed based on the obtained deformation field of the abdominal cavity in which the liver is located, and its registration map is obtained.

In 4614, the registration map of the floating image may be registered with a region in a first grayscale difference range in a reference image to obtain a third preliminary deformation field.

In some embodiments, the reference image refers to a pre-registration target image, which may also be referred to as a target image that has not been registered. For example, when the preoperative enhanced image is registered to the intraoperative scanning image, the reference image refers to the intraoperative scanning image for which no registration was performed. In some embodiments, the third preliminary deformation field may be a local deformation field. In some embodiments, operation 4614 may be performed as follows. A pixel grayscale calculation is performed on the registration map of the floating image and different regions of the reference image, respectively, to obtain corresponding grayscale values. The difference value between the grayscale of the registration map of the floating image and the grayscale of the corresponding region of the reference image is calculated. When the difference is in the first grayscale difference range, the registration map of the floating image and the corresponding region of the reference image are flexibly registered to obtain the third preliminary deformation field. In some embodiments, the difference being in the first grayscale difference range may indicate that a region of the registration map of the floating image is not much different from or is relatively small compared to a corresponding region in the reference image. For example, if the first grayscale difference is within a range from 0 to 150, the grayscale difference between the region Q1 in the registration map of the floating image and the same region in the reference image is 60, and the grayscale difference between the region Q2 in the registration map of the floating image and the same region in the reference image is 180. The difference between the region Q1 in the two images (i.e., the registration map of the floating image and the reference image) is not significant and the difference between the region Q2 is large, thus the registration is performed only on the region Q1 in the two images. In some embodiments, as shown in FIG. 16, a deformation field 3 (i.e., the third preliminary deformation field described above) is obtained by flexibly registering the registration map of the floating image to the regions of the reference image that conform to the first grayscale difference range (regions with less differences).

In 4615, a fourth preliminary deformation field of the full image may be determined based on the third preliminary deformation field.

In some embodiments, based on the third preliminary deformation field, interpolation is performed to obtain the fourth preliminary deformation field of the full image. In some embodiments, the fourth preliminary deformation field may be a global deformation field. In some embodiments, the local third preliminary deformation field may be changed to the global fourth preliminary deformation field by this operation. For example, a deformation field 4 of the entire image size is determined by interpolation from deformation field 3 as shown in FIG. 16.

In 4616, a region in a second grayscale difference range may be registered based on the fourth preliminary deformation field to obtain a registration map of a final registration.

In some embodiments, the region in the second grayscale difference range may be a region with a larger grayscale difference when comparing the grayscale values of the registration map and the floating image. In some embodiments, a grayscale difference threshold may be set (e.g., a grayscale difference threshold of 150), and a region in which a difference between the grayscale value of the registration map of the floating image and the grayscale value of the reference image is less than the grayscale difference threshold is the region in the first grayscale difference range, and a region in which a difference between the grayscale value of the registration map of the floating image and the grayscale value of the reference image is greater than the grayscale difference threshold is the region in the second grayscale difference range.

In some embodiments, the registration map of the final registration may be an image obtained by deforming the floating image (e.g., the preoperative enhanced image) in a plurality of times based on at least one deformation field, which is ultimately at the same spatial position and anatomical position as the intraoperative scanning image. In some embodiments, as shown in FIG. 8, based on the fourth preliminary deformation field, the registration map of the final registration may be obtained by registering the region in the second grayscale difference range (i.e., where the grayscale difference is relatively large). For example, a region other than the spleen, which has a relatively large grayscale difference, is targeted for deformation by the deformation field 4 to obtain the final registration map.

In some embodiments, using the registration manner described in FIGs. 15-16, elements that are segmented in the floating image and are not segmented in the reference image (e.g., blood vessels within the target organ), may be mapped from the floating image to the reference image. Taking the example that the floating image is the preoperative enhanced image and the reference image is the intraoperative scanning image, the blood vessels within the target organ are segmented in the preoperative enhanced image and are not segmented in the intraoperative scanning image, and the blood vessels in the target organ may be mapped to the intraoperative scanning image by registration. It will be appreciated that the registration manner of FIGs. 15-16 may also be used for the registration of the non-interventional region in the fast segmentation mode and all vital organs in the fine segmentation mode, or similar results may be achieved only by the corresponding segmentation manner.

In 462, a spatial position of a corresponding element may be determined during the procedure based on spatial positions of at least a portion of the elements in the first target structure set in the registration deformation field and the preoperative enhanced image. In some embodiments, a spatial position of blood vessels (hereinafter referred to as blood vessels) within the target organ during the procedure may be determined based on the registration deformation field and the registered vessels within the target organ in the preoperative enhanced image.

In some embodiments, the spatial position of the blood vessels during the procedure may be determined based on the registration deformation field and the blood vessels within the preoperative enhanced image by the following equation.

$$\tilde{I}(\mathrm{x, y, z}) = I_Q((x, y, z) + u(x, y, z)). \qquad (1)$$

Where $I_Q$ denotes the preoperative enhanced image, $(x, y, z)$ denotes the three-dimensional spatial coordinates of the blood vessels, $u(x, y, z)$ denotes the registration deformation field from the preoperative enhanced image to the intraoperative scanning image, and $\tilde{I}(x,y,z)$ denotes the spatial position of the blood vessels in the intraoperative scanning image. In some embodiments, $u(x,y,z)$ may also be understood as an offset from the three-dimensional coordinates of an element in the floating image (e.g., the blood vessels within the target organ) to the three-dimensional coordinates in the registration map of the final registration.

Thereby, through the registration deformation field determined in operation 461, the blood vessels in the preoperative enhanced image may be deformed to generate the spatial position of the blood vessels during the procedure that is identical to the spatial position of the blood vessels in the preoperative enhanced image.

In some embodiments, the processing device may calculate a center point of the lesion based on the determined spatial position of the blood vessels during the procedure and the lesion (included in the second segmentation image of the intraoperative scanning image), and generate a safe region around the lesion and a potential needle entry region. In some embodiments, the safe area around the lesion and the potential area for needle entry may be determined based on the interventional region and the non-interventional region. In some embodiments, a baseline path from a percutaneous needle entry point to the center point of the lesion may be planned based on the potential needle entry region and basic obstacle avoidance constraints. In some embodiments, the basic obstacle avoidance constraints may include, but are not limited to, a needle entry angle of the path, a needle entry depth of the path, and a lack of intersection between the path and the blood vessels and vital organs.

In 470, an interventional path may be planned based on spatial positions of the third target structure set during the procedure, and a risk assessment may be performed based on the interventional path.

In some embodiments, spatial positions of the elements in the third target structure set (e.g., the target organ, the lesion, the blood vessels within the target organ, the non-interventional region, and all vital organs) may more fully and accurately reflect a current condition of the target object (e.g., the patient). The interventional path may be planned based on the spatial positions of the third target structure set so that surgical instruments (e.g., puncture needles) may efficiently avoid the blood vessels, the non-interventional region and/or all vital organs within the target organ to reach the lesion smoothly while minimizing procedure risks.

In some embodiments, the elements of the third target structure set may be selected based on a mode of planning the interventional path. In some embodiments, the elements of the third target structure set used to determine the risk assessment of the interventional path may be different when the modes of planning the interventional path are different. For example, in a fast planning mode, the elements in the third target structure set used to determine the risk assessment of the interventional path may include the blood vessels within the target organ and the non-interventional region. As another example, in the precise planning mode, the elements in the third target structure set used to determine the risk assessment of the interventional path may include the blood vessels within the target organ and all vital organs.

More descriptions regarding the risk assessment may be found in the relevant descriptions of FIG. 2 and will not be repeated here.

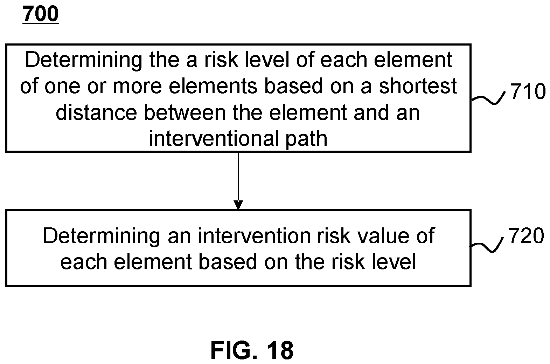

FIG. 18 is a flowchart illustrating an exemplary process for determining intervention risk values of one or more elements of a third target structure set under a fast planning mode according to some embodiments of the present disclosure.

In some embodiments, a process 700 for determining intervention risk values of one or more the elements of the third target structure set in a fast planning mode may include the following operations.

In 710, a risk level of each element of the one or more elements may be determined based on a shortest distance between the element and an interventional path.

In 720, an intervention risk value of each element may be determined based on the risk level.

In some embodiments, the element of the process 700 may include blood vessels and a non-interventional region within the target organ. Specifically, when the interventional path passes through the target organ in the third target structure set, the element in the process 700 may be the blood vessels within the target organ and the non-interventional region. At the same distance, the blood vessels within the target organ and the non-interventional region have different risk levels for the interventional path, corresponding to different risk values. The element in process 700 may be the non-interventional region when the interventional path does not pass through the target organ in the third target structure set. Thus, a shortest distance between the blood vessels within the target organ and the interventional path, and a shortest distance between the non-interventional region and the interventional path may be used to determine a risk level of a corresponding element, respectively, to determine a corresponding intervention risk value of the element.

From the above description, it can be seen that in the two cases that the interventional path passes through or does not pass through the target organ, elements that require calculation of the risk level and the risk value are different, calculation manners for risk values of elements of two different cases are respectively described below.

When the interventional path passes through the target organ, risk levels of the blood vessels in the target organ and the non-interventional region and intervention risk values need to be calculated. The intervention risk value of the blood vessels within the target organ is calculated as follows. A closest straight-line distance between the interventional path to the blood vessels is L1, when 0<L1<M1, the risk level is the highest (referred to as a first risk level), and a corresponding intervention risk value is a first intervention risk value. When M1<L1<N1, the risk level is a second risk level, and a corresponding intervention risk value is a second intervention risk value. When N1<L1<P1, the risk level is a third risk level, and a corresponding intervention risk value is a third intervention risk value. When L1>P1, the risk level and the intervention risk value are not considered. The first risk level is higher than the second risk level and the second risk level is higher than the third risk level. For example, when taking the values M1=5mm, N1=10mm and P1=15mm, the first intervention risk value corresponding to the first risk level may be 5 points. When 5<L1<=10, the second intervention risk value corresponding to the second risk level is 3 points. When 10<L1<=15, the third intervention risk value corresponding to the third risk level is 1 point. When L1>15, the risk level and intervention risk value are not considered (which may also be understood as an intervention risk value of 0 points).

The intervention risk value of the non-interventional region is calculated as follows. When a closest straight-line distance between the interventional path and the non-interventional region is L2, when 0<L2<A1, the risk level is the highest (referred to as a first risk level), and a corresponding intervention risk value is a first intervention risk value. When A1<L2<B 1, the risk level is a second risk level, and a corresponding intervention risk value is a second intervention risk value. When B1<L2<C1, the risk level is a third risk level, and a corresponding intervention risk value is a third intervention risk value. When L2>C1, the risk level and the intervention risk value are not considered. For example, when taking the values A1=3mm, B1=6mm, C1=10mm, when 0<L2<=3, the first intervention risk value corresponding to the first risk level may be 5 points. When 3<L2<=6, the second intervention risk value corresponding to the second risk level is 3 points. When 6<L2<=10, the third intervention risk value corresponding to the third risk level is 1 point. When L2>10, the risk level and intervention risk value are not considered (which may also be understood as an intervention risk value of 0 points).

M1>A1, N1>B1, and P1>C1, this is because when the blood vessels within the target organ are involved in the calculation of the distance, the blood vessels within the target organ are usually nearer to the interventional path, the distance between the blood vessels within the target organ and the interventional path is relatively strictly controlled. The non-interventional region is farther away from the interventional path, therefore the distance between the non-interventional region and the interventional path is relatively less controlled. For example, when the distance between the blood vessels within the target organ and the interventional path, and the distance between the non-interventional region and the interventional path, are both 5 mm, the distance of 5 mm is more acceptable for the non-interventional region, the intervention risk value is 3. However, the distance of 5 mm is riskier for the blood vessels within the target organ, the intervention risk value is 5.

When the interventional path does not pass through the target organ, only the risk level and the intervention risk value of the non-interventional region are calculated. In this case, the risk level and the intervention risk value of the non-interventional region are calculated in the same way as that of the non-interventional region when passing through the target organ and are not repeated here.

In some embodiments, the interventional path with a smallest total risk value is determined as an optimal interventional path by calculating a total risk value of at least one interventional path, because the smaller the total risk value, the lower the risk. In some embodiments, the total risk value may be obtained by accumulating intervention risk values of a plurality of interventional paths. In some embodiments, the optimal interventional path that minimizes the total risk value is utilized to plan the interventional path.

In the fast planning mode, since the fast segmentation mode does not need to segment all organs and tissues in the scene, but only needs to segment the non-interventional region, and extract the position of the blood vessels (and the lesion) in the target organ that is not obvious in the intraoperative scanning image through the registration, when planning the interventional path, it is only necessary to bypass the non-interventional region and make the interventional path go directly to the lesion, which improves the efficiency of interventional planning and interventional procedures.

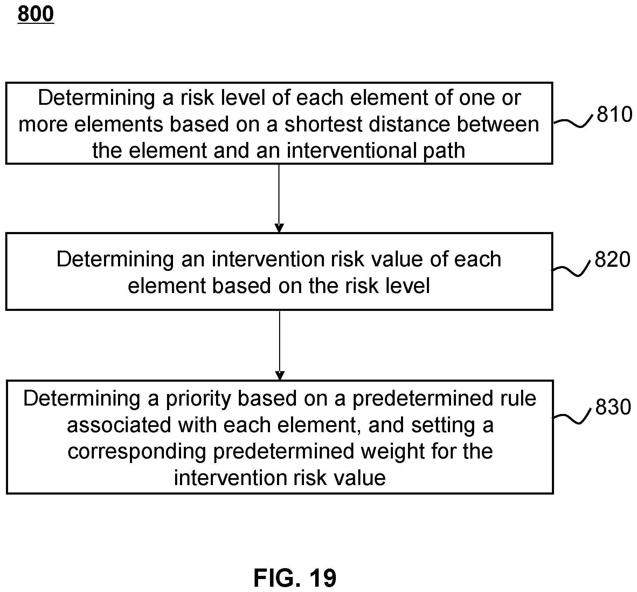

FIG. 19 is a flowchart illustrating an exemplary process for determining intervention risk values of one or more elements of a third target structure set under a precise planning mode according to some embodiments of the present disclosure.

In some embodiments, the process 800 for determining the intervention risk values of one or more elements of the third target structure set in the precise planning mode may include the following operations.

In 810, a risk level of each element of the one or more elements may be determined based on a shortest distance between the element and an interventional path.

In 820, an intervention risk value of each element may be determined based on the risk level.

In 830, a priority may be determined based on a predetermined rule associated with each element, and a corresponding predetermined weight for the intervention risk value may be set.

In some embodiments, elements in process 800 may include blood vessels and all vital organs within the target organ. Specifically, the elements in process 800 may be blood vessels within the target organ and all vital organs when the interventional path passes through the target organ in the third target structure set. When the interventional path does not pass through the target organ in the third target structure set, the elements in process 800 may be all vital organs. The predetermined rule may be used to characterize a non-interventional importance of different elements for the planned interventional path. For example, in a planned interventional path, the blood vessels within the target organ, and each of all vital organs, have a different non-interventional importance for the planned interventional path. Different elements have different priorities under the predetermined rule. In some embodiments, each element may be prioritized based on the predetermined rule associated with the element.

In some embodiments, in operation 830, determining the priority based on the predetermined rule associated with each element, and setting the corresponding predetermined weight for the intervention risk values may be implemented as follows. Based on the priority of each element of the one or more elements, the corresponding predetermined weight is set. In some embodiments, the priority of a segmentation region (i.e., the element) may be determined based on the non-interventional importance of the segmentation region (i.e., the element), e.g., the segmentation region such as the blood vessels, the vital organs, or the like, which must not be intervened, are set with a higher priority. In some embodiments, different predetermined weights may be assigned to elements of different priorities. In some embodiments, the higher the priority, the greater the corresponding predetermined weight, and the lower the priority, the smaller the corresponding predetermined weight. For example, the predetermined weight may be denoted by W, W ∈ 11, 0.8, 0.6}, where a larger predetermined weight (e.g., W is 1) mat be set when the priority is higher, and a smaller predetermined weight (e.g., W is 0.6) may be set when the priority is lower.

When the interventional path passes through the target organ, risk levels and the intervention risk values of the blood vessels within the target organ and all vital organs are calculated. The risk level and the intervention risk value of the blood vessels within the target organ are calculated in the same manner as in the fast planning mode and are not repeated here. The risk level and the intervention risk value of a vital organ may be calculated as follows. A risk level and an intervention risk value of a segmentation organ region are determined based on a closest distance between the interventional path and its neighboring vital organ (i.e., a distance between a needle pathway and a point of the neighboring organ closest to the needle pathway). In some embodiments, the risk level and the intervention risk value of the segmentation organ region may be determined based on whether the closest distance between the needle pathway and its neighboring non-interventional organ is within a set threshold. In some embodiments, the set threshold may be determined utilizing a plurality of set constant thresholds, such as denoted by X, Y, and Z, where X<Y<Z. The closest distance between the interventional path and its neighboring non-interventional organ may be denoted as L3, and the intervention risk value may be denoted as R. When it is determined that the intervention path passes through the organ, the planned intervention path immediately becomes invalid, and there is no need to reevaluate the intervention risk value. When 0<L3<X, the risk level is high, and the intervention risk value R is set to a. When X<L3<Y, the risk level is medium, and the intervention risk value is set to b. When Y<L3<Z, the risk level is low, and the intervention risk value R is set to c. When L3>Z, the risk may be ignored and the intervention risk value is set to 0, wherein a>b>c. In some embodiments, when the interventional path passes through the target organ, a corresponding priority may be determined based on the predetermined rules associated with the blood vessels within the target organ and all vital organs, and different weights may be assigned to the intervention risk values of the blood vessels and all vital organs with different priorities.

When the interventional path does not pass through the target organ, only the risk level and the intervention risk value of the vital organ are calculated. In this case, the risk level and the intervention risk value of the vital organ are calculated in the same way as when the interventional path passes through the target organ and are not repeated here. In some embodiments, when the interventional path does not pass through the target organ, a corresponding priority may be determined based on the predetermined rule associated with all vital organs, and intervention risk values of all vital organs with different priorities may be assigned with different weights.

In some embodiments, planning the interventional path based on the intervention risk value may be implemented as follows. A weighted risk value of at least one interventional path is calculated. The interventional path with the smallest weighted risk value may be determined as the optimal interventional path. In some embodiments, the weighted risk value may be obtained by weighting intervention risk values of the plurality of interventional paths. In some embodiments, the optimal interventional path that minimizes the weighted risk value is utilized to plan the interventional path. In some embodiments, the weighted risk value may be denoted as F. The weighted risk value F may be calculated by the following equation (2).

$$\mathrm{F} = \sum S * W \qquad (2)$$

When the weighted risk value F is smaller, the further away the needle path is from the vital organs and blood vessels, the lower the risk.

In the precise planning mode, when planning the interventional path (e.g., using a puncture needle, etc.), since contours of the target organ and other tissues in the scene may set up with the priority of intervention (e.g., puncture, etc.) according to the intervention risk value, and a reasonable interventional path is planned from an intervention point (e.g., an entry point, etc.) to the lesion, which is possible to avoid the non-interventional region with a high priority (e.g., blood vessels, vital organs, etc.) and obtain a potential needle entry space, thus improving the efficiency of intervention planning and its interventional procedures.

It should be noted that under the above two modes (i.e., the fast planning mode and the precise planning mode), the magnitude of the intervention risk values corresponding to different risk levels may be set according to the actual situation, and is not limited in the present disclosure.

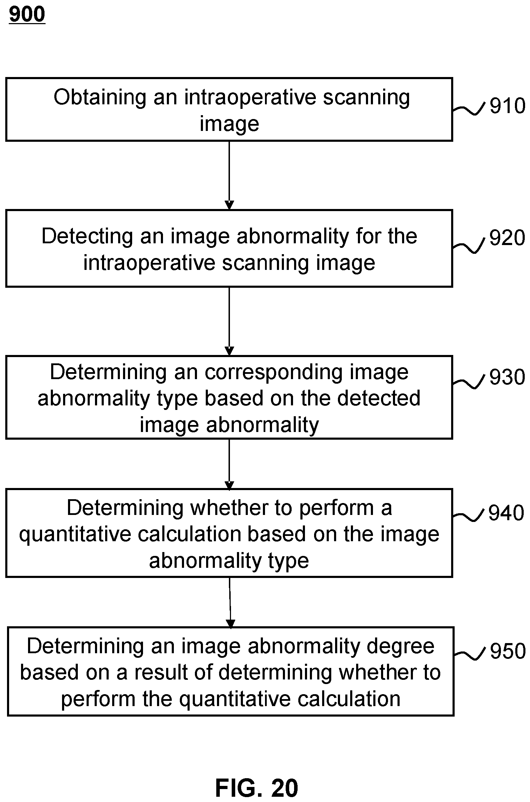

FIG. 20 is a flowchart illustrating an exemplary process for detecting an image abnormality according to some embodiments of the present disclosure.

In some embodiments, process 900 for detecting an image abnormality may include the following operations.

In 910, an intraoperative scanning image may be obtained.

In 920, an image abnormality may be detected for the intraoperative scanning image.

In 930, an image abnormality type may be determined based on the detected image abnormality.

In 940, whether to perform a quantitative calculation may be determined based on the image anomaly abnormality type.

In 950, an image abnormality degree may be determined based on a result of determining whether to perform the quantitative calculation.

In some embodiments, a description of the manner of obtaining the intraoperative scanning image may be found in related descriptions of FIGs. 2-5 and will not be repeated here. In some embodiments, the image abnormality may include a non-compliant portion of image data where complications exist. In some embodiments, the complications may include bleeding, pneumothorax, effusion, or the like.

In some embodiments, the image abnormality may be detected using a generative adversarial network approach modeled by deep learning after a comparison of normal data during modeling. In some embodiments, at least one of thresholding, image segmentation, and other manners may be used to detect the image abnormality. In some embodiments, the thresholding may be implemented in the following manner. Since feedback of different complications on the image is inconsistent, and pneumothorax, hemorrhage, effusion, or the like have varying ranges of pixel value distributions in the image, it is determined which complication the pixel value of the abnormal region belongs to by setting a pixel threshold. In some embodiments, the image segmentation may be implemented in the following manner. After obtaining the image abnormality, the abnormality is segmented using a deep learning algorithm, and pixels of the region in which the pixel abnormality is located are categorized, and it is determined which complication the pixel belongs to. If it is a non-complication, the procedure process may continue. Conversely, complications such as bleeding, effusion, pneumothorax, etc., may be quickly recognized and judged.

In some embodiments, the procedure process differs when the image abnormality type is different. For example, when the image abnormality type is a pneumothorax, an alarm alert may be sent to the operator and the procedure process ends. For example, when the image abnormality type is bleeding or effusion, the amount of bleeding or the amount of effusion may be quantitatively calculated, and it may be determined whether the procedure process is to be continued or to be ended based on results of the quantitative calculation. In some embodiments, the amount of bleeding or the amount of effusion corresponding to the region with the bleeding or effusion may be calculated based on an area percentage of the image. In some embodiments, it is possible to determine whether the amount of bleeding or the amount of effusion exceeds a predetermined threshold (e.g., a predetermined blood volume threshold, a predetermined effusion volume threshold), and when the predetermined threshold is not exceeded, a small amount of bleeding or effusion may not affect the process of the interventional procedure, and the procedure continues to be performed, and continuous observation may be performed. When the amount of bleeding or the amount of effusion exceeds the predetermined threshold, a safety issue arises, at this time, a reminder message may be sent to the doctor.

In some embodiments, a judgment result of the quantitative calculation determines an image abnormality degree. For example, a high image abnormality degree may be determined when the amount of bleeding or the amount of effusion exceeds the predetermined threshold, and a low image abnormality degree may be determined when the amount of bleeding or the amount of effusion does not exceed the predetermined threshold.

In some embodiments, a corresponding alarm prompt for the image abnormality degree may be sent based on the image abnormality type and the image abnormality degree. For example, when the image abnormality type is a pneumothorax, the operator may be sent with an alert message to stop the intervention. In some embodiments, when the image abnormality type is bleeding or effusion, different alarm prompts may be sent based on the image abnormality degree of the bleeding or effusion. For example, when the image abnormality degree is high, the alert message may prompt the operator to stop intervention. For example, when the image abnormality degree is low, an alert message that the operator may intervene and perform continuous observation may be sent.

Through the image abnormality detection, the complications that may occur at any time during the procedure and after the procedure may be effectively detected, avoiding the occurrence of the dangerous complications, and even in the case of complications, the doctor may be promptly reminded to stop the procedure in time for favorable treatment, improving the safety of interventional procedure.

FIG. 21 is a flowchart illustrating an exemplary process for postoperative assessment according to some embodiments of the present disclosure.

In some embodiments, the process 1000 of the postoperative assessment may include the following operations.

In 1010, a planned interventional path and an actual interventional path may be registered to the intraoperative scanning image.

In 1020, a deviation of the actual interventional path from the planned interventional path may be determined.

In 1030, whether the deviation has an intersection with a particular element in the third target structure set of the intraoperative scanning image may be determined.

In 1040, postoperative feedback information may be determined based on a determination result.

In some embodiments, the planned interventional path may be obtained based on a preoperative enhanced image and the intraoperative scanning image. The actual interventional path may be obtained based on a postoperative scanning image. In some embodiments, the postoperative scanning image refers to an image of a target object (e.g., a patient, etc.) scanned by a medical scanning device after an interventional procedure. More descriptions regarding obtaining the postoperative scanning image may be found herein above (e.g., FIG. 2), which will not be repeated here. The particular element in the third target structure set of the intraoperative scanning image may be a fourth target structure set. That is to say, in the fast planning mode, the particular element is the non-interventional region. In the precise planning mode, the particular element is all external vital organs or tissues.

In some embodiments, the planned interventional path and the actual interventional path may be registered to the intraoperative scanning image and a registration calculation may be performed to obtain a registration deformation field. In some embodiments, the registered interventional path may be displayed, and a difference calculation may be performed between the registered actual interventional paths and the planned interventional path. If there is a deviation between the actual interventional path and the planned interventional path, the deviated portion is extracted, and it is determined whether there is an intersection of the deviation with the non-interventional region or all vital organs or tissues of the intraoperative scanning image. If the intersection is not an empty set, it is proved that the actual interventional path may pass through the non-interventional region or all vital organs or tissues, which may affect the parenchyma, and at this time, it may be determined that the corresponding postoperative feedback information is a reminder message sent to the clinician. If the intersection is an empty set, the corresponding postoperative feedback information is determined to be no reminder. If there is no difference between the actual interventional path and the planned interventional path, the corresponding postoperative feedback information is determined to be no reminder.

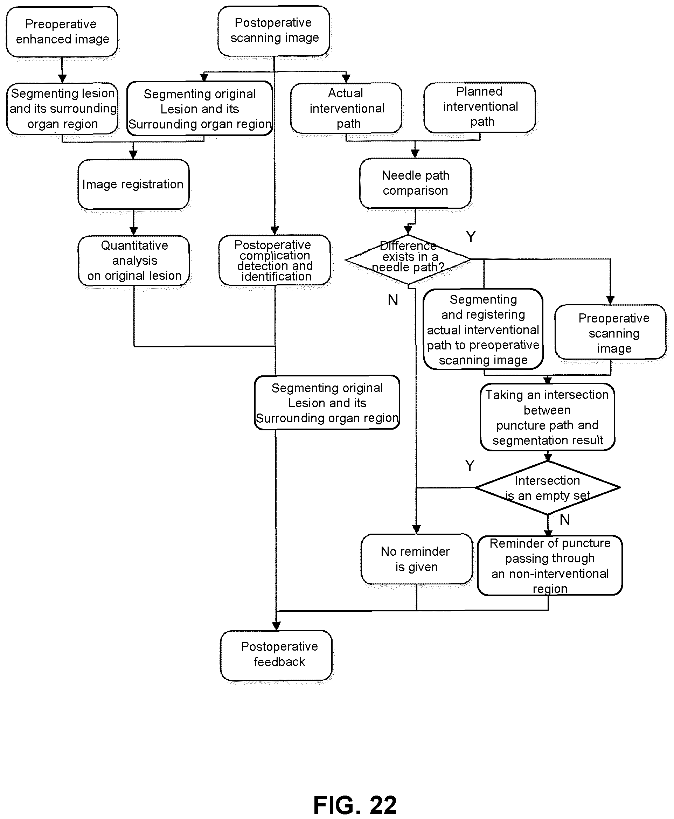

FIG. 22 is a flowchart illustrating an exemplary process for postoperative assessment according to some embodiments of the present disclosure.

In some embodiments, after obtaining the preoperative enhanced image and the postoperative scanning image, the lesion (original lesion) and its surrounding organ region are segmented, a region of interest of the region is intercepted for registration, so that positions of the preoperative lesion corresponds to the postoperative region of the original lesion, and then they are merged and displayed to facilitate the doctor to analyze the results of the procedure. In some embodiments, after obtaining segmentation results of a lesion and an original lesion region, the region is intercepted according to the segmentation results, so that, on the one hand, area of the lesion that changes in the postoperative period may be calculated for evaluating the efficiency of the procedure, on the other hand, pixels of the region may be analyzed to determine whether there is still a lesion present and the area of the lesion. In some embodiments, based on the postoperative scanning image, an image abnormality detection, i.e., a postoperative complication detection and identification, may be performed, in a manner described in FIG. 20, and will not be repeated here. In some embodiments, based on the postoperative scanning image, the actual interventional path may be extracted using over-threshold segmentation and deep learning, etc., to register the actual interventional path with the planned interventional path (i.e., needle path comparison), and to determine whether there is a change in order to assess the impact caused by the change and to realize accurate assessment. Specifically, when there is a deviation between the actual interventional path and the planned interventional path, the deviation of the actual interventional path from the planned interventional path is determined, and it is determined whether the deviation has the intersection with the particular element in the third target structure set of the intraoperative scanning image (i.e., the fourth target structure set). If the intersection is an empty set, no reminder is given. If the intersection is not an empty set, it indicates that the interventional path passes through the fourth target structure set, and the reminder message may be sent to the clinician. When there is no deviation between the actual interventional path and the planned interventional path, no reminder is given.

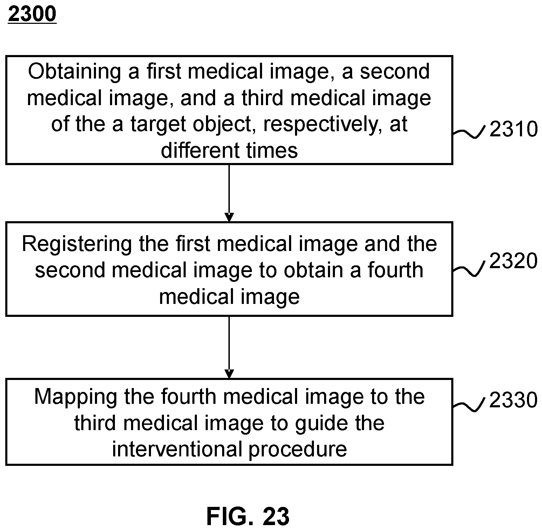

FIG. 23 is a flowchart illustrating an exemplary process for guiding an interventional procedure according to some embodiments of the present disclosure. In some embodiments, process 2300 may be performed by the processing device 140. For example, the process 2300 may be stored in a storage device (e.g., the storage device 150 and a storage unit of the processing device 140) in the form of a program or instruction, and the process 2300 may be implemented when the processor executes the program or instruction. In some embodiments, the process 2300 may be accomplished utilizing one or more additional operations not described below, and/or not by one or more operations discussed below.

In 2310, a first medical image, a second medical image, and a third medical image of a target object may be respectively obtained at different times.

In some embodiments, the processing device may obtain the first medical image, the second medical image, and the third medical image of the target object at different times through the medical scanning device. In some embodiments, the processing device may obtain the first medical image, the second medical image, the third medical image of the target object from the medical scanning device 110, the storage device 150, the storage unit of the processing device 140, or the like.

In some embodiments, the first medical image, the second medical image, and the third medical image may be obtained using a computed tomography (CT) device.

The first medical image may be a preoperative enhanced image or a preoperative plain scanning image. In some embodiments, the first medical image may be obtained before an interventional procedure. The period before the interventional procedure may be a certain period before the interventional procedure being performed, such as the first hour, the first two hours, the first five hours, the first day, the first two days, the first week, or the like. In some embodiments, the first medical image may be obtained at the time of the first visit of target object to the clinic, at the time of a routine physical examination, at the end of a previous interventional procedure.

The second medical image may be an intraoperative real-time image. In some embodiments, the second medical image is obtained during the interventional procedure and before the puncture is performed. The period during the interventional procedure and before the puncture being performed may be a preparation time before needle entry. For example, the second medical image may be obtained at a time of localization, a time of sterilization, a time of local anesthesia, or the like. As another example, the second medical image may be a first frame of the intraoperative real-time image.

The third medical image may be a real-time image generated during the interventional procedure. In some embodiments, the third medical image is obtained during a puncture execution process. The puncture execution process refers to a process of entering the needle from the skin, following a puncture path into a target region, completing the maneuver in the target region, and exiting the needle.

In some embodiments, the first medical image, the second medical image, and the third medical image may be obtained by different imaging devices. For example, the first medical image may be obtained by an imaging device in an imaging room, and the second medical image and the third medical image may be obtained by an imaging device in an operating room. In some embodiments, image parameters (e.g., an image range, accuracy, contrast, a gray scale, a gradient, etc.) of the first medical image, the second medical image, and the third medical image may be the same or different. For example, a scanning range of the first medical image may be greater than scanning ranges of the second medical image and the third medical image, or the accuracy of the second medical image and the third medical image may be higher than the first medical image.

More descriptions regarding obtaining the first medical image, the second medical image, and the third medical image may be found in the relevant descriptions of operation 210 of FIG. 2 and will not be repeated here.

In 2320, the first medical image and the second medical image may be registered to obtain a fourth medical image.

In some embodiments, the fourth medical image may include registered interventional procedure planning information. In some embodiments, the first medical image is obtained before the interventional procedure, with relatively ample time for acquisition and image processing, and a scanning range of the first medical image is relatively large, and the slices are thicker, e.g., including a large number of slices encompassing all the relevant tissues and/or organs. Planning the puncture path on a more comprehensively informative first medical image facilitates the accuracy of subsequent interventional procedure guidance.

In some embodiments, the second medical image is obtained during the interventional procedure, before the puncture execution process, with a relatively tight time for acquisition and image processing, and the scanning range of the second medical image is relatively small and slices are thin, which, for example, may include only 4 to 10 slices encompassing surrounding the needle tip. It will be appreciated that the fourth medical image obtained by registering the first medical image and the second medical image may include the registered interventional procedure planning information.

Descriptions regarding registering the first medical image and the second medical image may be found in the relevant descriptions of operation 220 of FIG. 2 and will not be repeated here.

In 2330, the fourth medical image may be mapped to the third medical image to guide the interventional procedure.

Descriptions regarding operation 2330 may be found in FIG. 2 and will not be repeated here.

FIG. 24 is a schematic diagram illustrating an exemplary guiding method for an interventional procedure according to some embodiments of the present disclosure.

In some embodiments, the processing device monitors breathing of the target object via a respiratory gating device. For example, as shown in FIG. 24, the respiratory gating device may obtain a respiratory amplitude point A where the target object is located when obtaining the first medical image. During the interventional procedure and before the puncture, the respiratory gating device may monitor the breathing of the target object and cause the medical scanning device to obtain the second medical image when the target object is at a respiratory amplitude point A'. The processing device obtains a puncture planning information image by processing the first medical image and obtains first deformation information by first registration. The processing device applies the first deformation information to the puncture planning information image to obtain the fourth medical image, the fourth medical image including puncture planning information after the first registration.

During puncture execution process, the target object may self-control (e.g., hold his/her breath) the respiratory amplitude to the same or similar respiratory amplitude. Or the processing device may monitor the target object's breathing amplitude via the respiratory gating device. When the target object adjusts the breathing to the third respiratory amplitude point A", the medical scanning device collects the third medical image, and the processing device maps the fourth medical image to the third medical image to guide the interventional procedure. If the respiratory gating device detects a significant deviation in the respiratory amplitude, the processing device may give a prompt and/or interrupt the puncture. When the target object is adjusted to the same or similar respiratory amplitude, the puncture continues.

Obtaining the first medical image, the second medical image, and the third medical image at the same or nearly the same respiratory amplitude point allows for less movement of the organs and tissues between images caused by respiratory motion, which is conducive to improving the accuracy of preoperative planning.

In some embodiments of the present disclosure, by combining planning results of the large-view thin-slice image scanned before the interventional procedure with the intraoperative real-time image, i.e., the real-time image is utilized to display in real-time the state of the puncture site of the patient, and a preinterventional procedure detailed planning result and detailed information are utilized to avoid high-risk regions and reduce procedure risk. And the intraoperative real-time puncture process is based on the position of the puncture needle tip in the center of the field of view, the puncture needle is punctured to the lesion along the puncture planned path, the CT bed shifts or moves a detector to update the scanning range, to obtain a real-time scanning image, which guides the puncture process, improving procedure efficiency and reducing procedure risk.

FIG. 25 is another schematic diagram illustrating an exemplary process for guiding an interventional procedure according to some other embodiments of the present disclosure.

In some embodiments, the processing device may monitor the breathing of the target object without the aid of the respiratory gating device. As shown in FIG. 25, the processing device obtains the first medical image, the second medical image, and the third medical image of the target object at different times. The processing device obtains the puncture planning information image by processing the first medical image and obtains the first deformation information by first registration. The processing device applies the first deformation information to the puncture planning information image to obtain the fourth medical image, the fourth medical image including puncture planning information obtained after the first registration.

The processing device registers the second medical image and the third medical image a second time to obtain the second deformation information and applies the second deformation information to the fourth medical image to obtain a fifth image, the fifth image including puncture planning information after the second registration. The processing device maps the fifth image to the third medical image to guide the interventional procedure.

Because the second medical image and the third medical image are both data at slice-less layers, the second registration has a small computational cost, and the registration may be realized in a shorter time period after obtaining the third medical image during the procedure, reducing the risk of procedure.

Embodiments of the present disclosure also provide a surgical robot including a robotic arm to perform the interventional procedure, and a control system. The control system includes at least one processor and at least one storage medium. The storage medium stores operation instructions adapted to cause the processor to perform the following operations. The first medical image, the second medical image, and the third medical image of the target object are respectively obtained at different times. The first medical image and the second medical image are registered to obtain the fourth medical image, the fourth medical image including the registered puncture planning information. The fourth medical image is mapped to the third medical image, respectively, to guide the interventional procedure.

Embodiments of the present disclosure also provide a surgical robot including a robotic arm to perform the interventional procedure, and a control system. The control system includes at least one processor and at least one storage medium. The storage medium stores operation instructions adapted to cause the processor to perform the following operations. The first medical image, the second medical image, and the third medical image of the target object are respectively obtained at different times. The first medical image and the second medical image are first registered to obtain the first deformation information and the fourth medical image, the fourth medical image including the registered puncture planning information. The second medical image and the third medical image are registered at a second time to obtain the second deformation information. The second deformation information is applied to the fourth medical image to obtain the fifth image, the fifth image including puncture planning information obtained after the second registration. The fifth image is mapped to the third medical image to guide the interventional procedure.

It should be noted that the foregoing descriptions with respect to the respective processes are for the purpose of exemplification and illustration only and do not limit the scope of application of the present disclosure. For a person skilled in the art, various corrections and changes may be made to the individual processes under the guidance of the present disclosure, for example, by adding a storage step, or the like.

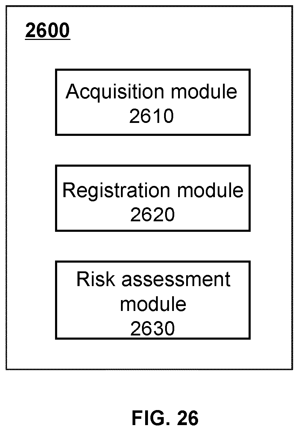

FIG. 26 is a schematic diagram illustrating exemplary modules of a medical image processing system for an interventional procedure according to some embodiments of the present disclosure. As shown in FIG. 26, the system 2600 may include an acquisition module 2610, a registration module 2620, and a risk assessment module 2630.

The acquisition module 2610 is configured to obtain a first medical image of a target object before an interventional procedure and a second medical image of the target object during the interventional procedure.

The registration module 2620 is configured to register the second medical image and the first medical image to obtain a registration result.

The risk assessment module 2630 is configured to determine interventional procedure planning information of the target object at least based on the registration result, perform an interventional procedure risk assessment based on the interventional procedure planning information, and obtain a risk assessment result corresponding to the interventional procedure planning information.

It should be noted that more technical details regarding the acquisition module 2610, the registration module 2620, and the risk assessment module 2630, which perform the corresponding processes or functions to realize interventional procedure image assistance, may be found in FIGs. 1-25 described in any one of the embodiments for interventional procedure medical image processing manner, which will not be repeated herein.

The above description of the medical image processing system 2600 for the interventional procedure is for illustrative purposes only and is not intended to limit the scope of the present disclosure. For a person of ordinary skill in the art, the application of the above method and system may be improved and altered in various forms and details without departing from the principles of the present disclosure. However, these changes and modifications do not depart from the scope of the present disclosure. In some embodiments, the medical image processing system 2600 for the interventional procedure may include one or more other modules. For example, the medical image processing system 2600 for interventional procedure 2600 may include a storage module to store data generated by the modules of the medical image processing system 2600 for the interventional procedures. In some embodiments, the acquisition module 2610, the registration module 2620, and the risk assessment module 2630 of FIG. 26 may be different modules in a single system, or a single module may implement the functions of two or more modules. For example, the individual modules may share a common storage module, and the individual modules may each have a respective storage module. The features, structures, methods, and other characteristics of the exemplary embodiments described in the present disclosure may be combined in a variety of ways to obtain additional and/or alternative exemplary embodiments. For example, the processing device 140 and the medical scanning device 110 may be integrated into a single device. Such morphs are within the scope of protection of the present disclosure.

Some embodiments of the present disclosure also provide a medical image processing device for interventional procedures, including a processor. The processor is configured to perform a medical image processing method for the interventional procedure as described in any of the embodiments, as described in FIGs. 1-25, and will not be repeated herein.

Some embodiments of the present disclosure further provide a non-transitory computer-readable storage medium storing computer instructions. When a computer reads the computer instructions, the computer performs the medical image processing method for the interventional procedure in any of the above embodiments. Detailed descriptions may be found in FIGs. 1-25, which will not be repeated herein.

The medical image processing method, system and device for an interventional procedure and the non-transitory computer-readable storage medium provided by embodiments of the present disclosure have at least the following beneficial effects.

(1) First of all, considering characteristics of the preoperative enhanced image with good rendering effect on targets such as blood vessels and lesions, and the advantages of the intraoperative scanning image close to the real situation of the patient, the segmentation process adopts the optimization of segmentation manner from coarse to fine combined with deep learning, which supports accurate organ segmentation through precise organ localization, and improves the efficiency of segmentation and the robustness of image processing.

(2) Second, by adopting the soft connected component analysis manner in the coarse segmentation stage, the target structure set region is accurately reserved while the false positive region is effectively excluded, which firstly improves the accuracy of the element localization in the coarse localization stage and directly contributes to the subsequent reasonable extraction of the bounding box of the localization information of the element mask, thus enhancing the efficiency of the segmentation.

(3) Third, for the unfavorable situation that the coarse localization is inaccurate but not invalid in the coarse segmentation stage, utilizing the adaptive sliding window calculation and the corresponding sliding window operation, which is able to fill in the missing portion of the localization region, and may automatically plan and execute reasonable sliding window operations, reducing the dependence on the coarse localization result in the fine segmentation stage, and improving the segmentation accuracy while keeping the segmentation time and the computational resources not significantly increased.

(4) Fourth, even when the coarse localization is invalid, the element mask may be accurately localized based on the predetermined localization coordinates of the element, which not only improves the segmentation accuracy, but also reduces the segmentation time, reduces the amount of segmentation calculation, and further improves the segmentation efficiency.

(5) Then, since the overall workflow of the target structure set segmentation fully considers a plurality of unfavorable scenarios that reduce the accuracy of target structure segmentation, it makes it applicable to the effective implementation of different kinds of target structure set segmentation tasks with high segmentation accuracy and segmentation robustness.

(6) Moreover, by synthesizing the respective advantages of the preoperative enhanced image and intraoperative scanning image, the fast segmentation mode and the fine segmentation mode (the planning mode is only for intraoperative scanning images) is set up, and different path planning schemes are determined according to the selected planning mode. Under the fast segmentation mode, the planning speed is fast and the time is short under the fine segmentation mode, the planning path is more selective and with high robustness, which provides stronger processing applicability and guarantees the stability of the system and the safety of the intervention, enabling the preoperative planning to reach a higher accuracy, so as to better assist the accurate implementation of the corresponding puncture path during the procedure and obtain a more ideal procedure effect.

(7) Further, two fully automated modes are provided for interventional procedure planning. In the precise planning mode, when performing the interventional (e.g., puncture needle, etc.) path planning, the efficiency of interventional planning and its interventional procedure is improved since reasonable paths may be planned, which may avoid blood vessels and vital organs with higher priorities and obtain potential space for needle entry. In the fast planning mode, as the interventional path may bypass the non-interventional region and go directly to the lesion in the fast segmentation mode, which also improves the efficiency of interventional planning and interventional procedures

(8) In addition, the optimal interventional path may be planned efficiently, accurately, and automatically, and the interventional path risk may be analyzed, providing good preoperative planning guidance for the interventional procedures. During the procedure, real-time detection and identification of complications are provided, which further enhances the safety of the intervention process. Additionally, the workflow also realizes the postoperative evaluation function, which may assist the operator to accurately evaluate the procedure process and the procedure result, thus improving the efficiency and safety of the procedure.

(9) Obtaining images at the same or similar respiratory amplitude point allows for less movement of organ tissues between images caused by respiratory movements, which contributes to the accuracy of preoperative planning.

(10) Performing high-precision registration before puncture execution avoids or reduces computational stress after the start of puncture execution and reduces the duration of puncture execution.

(11) Prolonged breath holding of the patient is also avoided, which improves the patient's experience.

(12) A large field of view of guided image is displayed, real-time images and planned images are displayed in different ways, thus the interventional procedure is clearly guided.

Having thus described the basic concepts, it may be rather apparent to those skilled in the art after reading this detailed disclosure that the foregoing detailed disclosure is intended to be presented by way of example only and is not limiting. Although not explicitly stated here, those skilled in the art may make various modifications, improvements, and amendments to the present disclosure. These alterations, improvements, and amendments are intended to be suggested by this disclosure and are within the spirit and scope of the exemplary embodiments of the present disclosure.

Moreover, certain terminology has been used to describe embodiments of the present disclosure. For example, the terms "one embodiment," "an embodiment," and/or "some embodiments" mean that a particular feature, structure, or feature described in connection with the embodiment is included in at least one embodiment of the present disclosure. Therefore, it is emphasized and should be appreciated that two or more references to "an embodiment" or "one embodiment" or "an alternative embodiment" in various portions of the present disclosure are not necessarily all referring to the same embodiment. In addition, some features, structures, or characteristics of one or more embodiments in the present disclosure may be properly combined.

Furthermore, the recited order of processing elements or sequences, or the use of numbers, letters, or other designations, therefore, is not intended to limit the claimed processes and methods to any order except as may be specified in the claims. Although the above disclosure discusses some embodiments of the invention currently considered useful by various examples, it should be understood that such details are for illustrative purposes only, and the additional claims are not limited to the disclosed embodiments. Instead, the claims are intended to cover all combinations of corrections and equivalents consistent with the substance and scope of the embodiments of the present disclosure. For example, although the implementation of various components described above may be embodied in a hardware device, it may also be implemented as a software only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. However, this disclosure does not mean that object of the present disclosure requires more features than the features mentioned in the claims. Rather, claimed subject matter may lie in less than all features of a single foregoing disclosed embodiment.

In some embodiments, the numbers expressing quantities or properties used to describe and claim certain embodiments of the present disclosure are to be understood as being modified in some instances by the term "about," "approximate," or "substantially." For example, "about," "approximate" or "substantially" may indicate $\pm20\%$ variation of the value it describes, unless otherwise stated. Accordingly, in some embodiments, the numerical parameters set forth in the written description and attached claims are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the present disclosure are approximations, the numerical values set forth in the specific examples are reported as precisely as practicable.

In closing, it is to be understood that the embodiments of the present disclosure disclosed herein are illustrative of the principles of the embodiments of the present disclosure. Other modifications that may be employed may be within the scope of the present disclosure. Thus, by way of example, but not of limitation, alternative configurations of the embodiments of the present disclosure may be utilized in accordance with the teachings herein. Accordingly, embodiments of the present disclosure are not limited to that precisely as shown and described.

**Claims**

1. A medical image processing system for an interventional procedure, comprising:
   a control system including at least one processor and at least one storage medium, the at least one storage medium storing operating instructions, wherein when executing the operating instructions, the at least one processor is directed to cause the system to perform operations including:

   obtaining a first medical image of a target object before the interventional procedure and a second medical image of the target object during the interventional procedure;
   registering the second medical image and the first medical image to obtain a registration result; and
   determining interventional procedure planning information of the target object at least based on the registration result, performing an interventional procedure risk assessment based on the interventional procedure planning information, and obtaining a risk assessment result corresponding to the interventional procedure planning information.

2. The system of claim 1, wherein the obtaining a first medical image of a target object before the interventional procedure and a second medical image of the target object during the interventional procedure includes:

   obtaining a preoperative enhanced image;
   obtaining a first medical image of a first target structure set by segmenting the first target structure set from the preoperative enhanced image;
   obtaining an intraoperative scanning image; and
   obtaining a second medical image of a second target structure set by segmenting the second target structure set from the intraoperative scanning image, wherein the first target structure set has an intersection with the second target structure set.

3. The system of claim 2, wherein:

   the registration result includes a spatial position of a third target structure set in the interventional procedure, and elements of the third target structure set are determined based on a mode of planning an interventional path; and
   at least one element in the third target structure set is included in the first target structure set and at least one element in the third target structure set is excluded from the second target structure set.

4. The system of claim 3, wherein the performing an interventional procedure risk assessment based on the interventional procedure planning information includes:

   determining intervention risk values of one or more elements of the third target structure set, each of the intervention risk values corresponding to one of the one or more elements; and
   performing the interventional procedure risk assessment based on the intervention risk values.

5. The system of claim 4, wherein the performing an interventional procedure risk assessment based on the interventional procedure planning information further includes:

   determining whether a planned interventional path in the interventional procedure planning information crosses a predetermined element in the third target structure set; and
   in response to a determination that the planned interventional path in the interventional procedure planning information crosses the predetermined element in the third target structure set, determining the intervention risk value of a predetermined risk object in the third target structure set.

6. The system of claim 4 or 5, wherein the determining intervention risk values of one or more elements of the third target structure set includes:

determining a risk level of each element of the one or more elements based on a shortest distance between the element and the planned interventional path; and
determining an intervention risk value of each element based on the risk level.

7. The system of claim 4 or 5, wherein the determining intervention risk values of one or more elements of the third target structure set includes:

determining a risk level of each element of the one or more elements based on a shortest distance between the element and the planned interventional path; and
determining an intervention risk value of each element based on the risk level; and
determining a priority based on a predetermined rule associated with each element, and setting a corresponding predetermined weight for the intervention risk value.

8. The system of claim 4, wherein the performing the interventional procedure risk assessment based on the intervention risk values includes:

determining a total risk value of at least one interventional path; and
determining an interventional path with a smallest total risk value as an optimal interventional path.

9. The system of claim 3, wherein:

the mode of planning the interventional path includes a fast planning mode or a precise planning mode, and
a ratio of a total volume of elements of the third target structure set in the fast planning mode to a total volume of the elements of the third target structure set in the precise planning mode is greater than a predetermined efficiency factor m.

10. The system of claim 9, wherein the predetermined efficiency factor m is related to a type of the interventional procedure.

11. The system of claim 1, wherein the operations further include:

obtaining an intraoperative scanning image;
detecting an image abnormality for the intraoperative scanning image;
determining an image abnormality type based on the detected image abnormality;
determining whether to perform a quantitative calculation based on the image abnormality type; and
determining an image abnormality degree based on a result of determining whether to perform the quantitative calculation.

12. The system of claim 10, wherein the operations further include:
providing an alarm indication corresponding to the image abnormality degree based on the image abnormality degree.

13. The system of claim 10, wherein the operations further include:

registering the planned interventional path obtained based on the preoperative enhanced image and the intraoperative scanning image, and an actual interventional path obtained based on a postoperative scanning image, to the intraoperative scanning image; and
determining postoperative feedback information based on a determination result of whether a deviation of the actual interventional path from the planned interventional path has an intersection with a particular element in the third target structure set of the intraoperative scanning image.

14. The system of claim 1, wherein the operations further include:
in response to the risk assessment result corresponding to the interventional procedure planning information satisfying a predetermined condition, guiding the interventional procedure based on the interventional procedure planning information satisfying the predetermined condition.

15. The system of claim 1, wherein the operations further include:

obtaining a third medical image of the target object in the interventional procedure; and
mapping the registration result to the third medical image to guide the interventional procedure.

16. The system of claim 15, wherein:

the first medical image is obtained when the target object is at a first respiratory amplitude point before the interventional procedure, the second medical image is obtained when the target object is at a second respiratory amplitude point during the interventional procedure and before a puncture procedure, and the third medical image is obtained when the target object is at a third respiratory amplitude point during the puncture procedure; and

a deviation of the second respiratory amplitude point from the first respiratory amplitude point is less than a predetermined value, and a deviation of the third respiratory amplitude point from the first respiratory amplitude point and/or the second respiratory amplitude point is less than the predetermined value.

17. The system of claim 15, wherein the registering the second medical image and the first medical image to obtain a registration result includes:

obtaining an interventional procedure planning information image based on the first medical image;

performing a first registration on the first medical image and the second medical image to obtain first deformation information; and

applying the first deformation information to the interventional procedure planning information image to obtain the registration result, wherein the interventional procedure planning information in the registration result is interventional procedure planning information obtained after the first registration.

18. The system of claim 17, wherein the operations further include at least one of:

displaying, outside a display range of the third medical image, image information of the registration result that lies outside the display range of the third medical image;

displaying information of the planed interventional path of the interventional procedure outside the display range of the third medical image; or

displaying image information within and outside the display range of the third medical image in different ways.

19. A medical image processing method for an interventional procedure, comprising:

obtaining a first medical image of a target object before the interventional procedure and a second medical image of the target object during the interventional procedure;

registering the second medical image and the first medical image to obtain a registration result; and

determining interventional procedure planning information of the target object at least based on the registration result, performing an interventional procedure risk assessment based on the interventional procedure planning information, and obtaining a risk assessment result corresponding to the interventional procedure planning information.

20. A medical image processing method for an interventional procedure, comprising:

obtaining a mode of planning an interventional path;

obtaining a preoperative enhanced image;

obtaining a first medical image of a first target structure set by segmenting the first target structure set from the preoperative enhanced image;

obtaining an intraoperative scanning image;

obtaining a second medical image of a second target structure set by segmenting the second target structure set from the intraoperative scanning image, wherein the first target structure set has an intersection with the second target structure set;

registering the first medical image and the second medical image, determining a spatial position of a third target structure set in the interventional procedure, selecting elements of the third target structure set based on the mode of planning the interventional path; and

obtaining a planned interventional path based on the spatial position of the third target structure set in the interventional procedure, and performing a procedure risk assessment based on the planned interventional path,

wherein:

at least one element in the third target structure set is included in the first target structure set and at least one element in the third target structure set is excluded from the second target structure set.

21. A guiding system for an interventional procedure, comprising:

a control system including at least one processor and at least one storage medium, wherein the at least one storage medium storing operating instructions, wherein when executing the operating instructions, the at least one processor is directed to cause the system to perform operations including:

obtaining a first medical image, a second medical image, and a third medical image of a target object, respectively, at different times;

registering the first medical image and the second medical image to obtain a fourth medical image, wherein the fourth medical image includes registered interventional procedure planning information; and
mapping the fourth medical image to the third medical image to guide the interventional procedure.

22. A medical image processing device for an interventional procedure, comprising a processor, wherein the processor is configured to execute operating instructions involved in the medical image processing system for the interventional procedure of any one of claims 1 to 18.

23. A non-transitory computer-readable storage medium storing computer instructions, wherein when a computer reads the computer instructions in the storage medium, the computer performs the method of claim 19.

<u>100</u>

**FIG. 1**

## 200

Obtaining a first medical image of a target object before an interventional procedure and a second medical image of the target object during the interventional procedure ⟶ 210

Registering the second medical image and the first medical image to obtain a registration result ⟶ 220

Determining interventional procedure planning information of the target object at least based on the registration result, performing an interventional procedure risk assessment based on interventional procedure planning information, and obtaining a risk assessment result corresponding to the interventional procedure planning information ⟶ 230

## FIG. 2

Obtaining a third medical image of a target
object in an interventional procedure ⌇∕310

Mapping the registration result to the third
medical image to guide the interventional
procedure ⌇∕320

## FIG. 3

Obtaining a pattern of a planned interventional path ~/410

Obtaining a preoperative enhanced image ~/420

Obtaining a first medical image of a first target structure set by segmenting the first target structure set from the preoperative enhanced image ~/430

Obtaining an intraoperative scanning image ~/440

Obtaining a second medical image of a second target structure set by segmenting the second target structure set from the intraoperative scanning image ~/450

Registering the first medical image and the second medical image and determining spatial locations of blood vessels and lesions in the procedure ~/460

Planning an interventional path based on spatial locations of a third target structure set during the procedure, and performing a risk assessment based on the interventional path ~/470

**FIG. 4**

<u>500</u>

Performing coarse segmentation on at least one element of a target structure set in a medical image ~/510

Obtaining a mask of at least one element ~/520

Determining positioning information of the mask ~/530

Segmenting the at least one element precisely based on the positioning information of the mask ~/540

**FIG. 5**

**530**

Determining a count of connected domains — 531

Determining area of a connected domain that meets a predetermined condition when the count of connected domains is greater than and equal to 2 — 532

Determining a largest connected domain meets the predetermined condition when a ratio of area of a largest connected domain among a plurality of connected domains to total area of the connected domains is greater than a first threshold M — 533

Determining a reserved connected domain includes at least the largest connected domain — 534

Determining location information of an element mask based on the reserved connected domain — 535

FIG. 6

**FIG. 7**

**FIG. 8**

540

| Performing a preliminary precise segmentation on at least one element | 541 |

↓

| Determining whether positioning information of an element mask is accurate | 542 |

No
↓

| Obtaining accurate positioning based on an adaptive sliding window | 543a |

Yes

| Outputting the preliminary precise segmentation result as a segmentation result | 543b |

FIG. 9

**FIG. 10**

**FIG. 11**

FIG. 12A

FIG. 12B

B1-2

C

B

A

B1

First direction

Second direction

**FIG. 12C**

B1-3

C

B

A

B1

First direction

Second direction

**FIG. 12D**

B1-1

B

A

B1-2

First direction

Second direction

B1

# FIG. 12E

**FIG. 13**

**460**

Determining registered deformation field by registering a first medical image and a second medical image ⟿ 461

Determining a spatial location of a corresponding element during a procedure based on spatial locations of at least a portion of elements in a first target structure set in a registered deformation field and a preoperative enhanced image ⟿ 462

**FIG. 14**

461

Determining a first preliminary deformation field based on a registration between elements ⟿ 4611

↓

Determining a second preliminary deformation field of the full image based on the first preliminary deformation field between elements ⟿ 4612

↓

Deforming a floating image based on the second preliminary deformation field of the full image to determine a registration map of the floating image ⟿ 4613

↓

Obtaining a third preliminary deformation field by registering the registration map of the floating image with a region of a first range of a first grayscale difference in a reference image ⟿ 4614

↓

Determining a fourth preliminary deformation field of the full image based on the third preliminary deformation field ⟿ 4615

↓

Obtaining a registration map of a final registration by registering a region in a second grayscale difference range based on the fourth preliminary deformation field ⟿ 4616

FIG. 15

FIG. 16

Preoperative enhanced image  Intraoperative scanning image

Segmentation

Organ contour A                    Organ contour B

**FIG. 17**

**700**

| Determining the a risk level of each element of one or more elements based on a shortest distance between the element and an interventional path | ～710 |

↓

| Determining an intervention risk value of each element based on the risk level | ～720 |

**FIG. 18**

800

Determining a risk level of each element of one or more elements based on a shortest distance between the element and an interventional path ~810

Determining an intervention risk value of each element based on the risk level ~820

Determining a priority based on a predetermined rule associated with each element, and setting a corresponding predetermined weight for the intervention risk value ~830

FIG. 19

900

Obtaining an intraoperative scanning image ~/910

Detecting an image abnormality for the intraoperative scanning image ~/920

Determining an corresponding image abnormality type based on the detected image abnormality ~/930

Determining whether to perform a quantitative calculation based on the image abnormality type ~/940

Determining an image abnormality degree based on a result of determining whether to perform the quantitative calculation ~/950

**FIG. 20**

<u>1000</u>

```
┌─────────────────────────────────────┐
│   Registering a planned interventional path      │
│   and an actual interventional path to an   │ ～⁄1010
│       intraoperative scanning image        │
└─────────────────────────────────────┘
                    │
                    ▼
┌─────────────────────────────────────┐
│     Determining a deviation of the actual       │
│     interventional path from the planned      │ ～⁄1020
│           interventional path              │
└─────────────────────────────────────┘
                    │
                    ▼
┌─────────────────────────────────────┐
│   Determining whether the deviation has an      │
│   intersection with a particular element in the   │ ～⁄1030
│       third target structure set of the        │
│        intraoperative scanning image         │
└─────────────────────────────────────┘
                    │
                    ▼
┌─────────────────────────────────────┐
│     Determining postoperative feedback        │ ～⁄1040
│   information based on a determination result   │
└─────────────────────────────────────┘
```

**FIG. 21**

Preoperative
enhanced image

Postoperative
scanning image

Segmenting lesion
and its surrounding
organ region

Segmenting original
Lesion and its
Surrounding organ region

Actual
interventional
path

Planned
interventional
path

Image registration

Needle path
comparison

Quantitative
analysis
on original lesion

Postoperative
complication
detection and
identification

Difference
exists in a
needle path?

Y

N

Segmenting
and registering
actual interventional
path to preoperative
scanning image

Preoperative
scanning
image

Segmenting original
Lesion and its
Surrounding organ region

Taking an intersection
between
puncture path and
segmentation result

Y

Intersection
is an empty set

N

No reminder
is given

Reminder of puncture
passing through
an non-interventional
region

Postoperative
feedback

FIG. 22

2300

Obtaining a first medical image, a second medical image, and a third medical image of the a target object, respectively, at different times    ∿ 2310

Registering the first medical image and the second medical image to obtain a fourth medical image    ∿ 2320

Mapping the fourth medical image to the third medical image to guide the interventional procedure    ∿ 2330

FIG. 23

**FIG. 24**

**FIG. 25**

**2600**

Acquisition module
2610

Registration module
2620

Risk assessment
module
2630

FIG. 26

Skin

Target organ

Puncture needle

B₁
C

Mapped high-risk tissue

Planned Interventional path

Scanning range of the image at T₁

Move the bed according to a change of needle tip location

Lesion

Target organ

Puncture needle

Mapped high-risk tissue

B₂

Planned

Skin

Interventional

Lesion

path

FIG. 27

Scanning range of the image at T₂

## INTERNATIONAL SEARCH REPORT

| International application No. |
|---|
| **PCT/CN2023/091895** |

| A. | CLASSIFICATION OF SUBJECT MATTER |
|---|---|
| | A61B34/10(2016.01)i; A61B17/34(2006.01)n |

According to International Patent Classification (IPC) or to both national classification and IPC

| B. | FIELDS SEARCHED |
|---|---|

Minimum documentation searched (classification system followed by classification symbols)

IPC:A61B34/-, A61B17/-; CPC:A61B2034/107

Documentation searched other than minimum documentation to the extent that such documents are included in the fields searched

Electronic data base consulted during the international search (name of data base and, where practicable, search terms used)

CJFD, CNTXT, DWPI, ENTXTC, ENTXT, bing学术, BING SCHOLAR: 联影, 何少文, 廖明哲, 张璟, 配准, 匹配, 手术, 前, 中, 后, 术前, 术中, 术后, 图像, 叠加, 方伟, 风险, 评估, 评价, 规划, 异常, 影像, intra, operative, intraoperative, preoperative, planning, navigation, evaluation

| C. | DOCUMENTS CONSIDERED TO BE RELEVANT |
|---|---|

| Category* | Citation of document, with indication, where appropriate, of the relevant passages | Relevant to claim No. |
|---|---|---|
| X | CN 112163987 A (SUZHOU INSTITUTE OF BIOMEDICAL ENGINEERING AND TECHNOLOGY, CHINESE ACADEMY OF SCIENCES et al.) 01 January 2021 (2021-01-01)<br>    description, paragraphs 55, 60-75, and 93-110 | 1-14, 19-20, 22-23 |
| Y | CN 112163987 A (SUZHOU INSTITUTE OF BIOMEDICAL ENGINEERING AND TECHNOLOGY, CHINESE ACADEMY OF SCIENCES et al.) 01 January 2021 (2021-01-01)<br>    description, paragraphs 55, 60-75, and 93-110 | 15-18, 21 |
| Y | CN 113057734 A (SHANGHAI MICROPORT MEDICAL ROBOT (GROUP) CO., LTD.) 02 July 2021 (2021-07-02)<br>    description, paragraphs 129-135 | 15-18, 21 |
| A | CN 103479430 A (JIANGSU MEILUN IMAGING SYSTEMS CO., LTD.) 01 January 2014 (2014-01-01)<br>    entire document | 1-23 |

☑ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **03 August 2023** | **07 August 2023** |

| Name and mailing address of the ISA/CN | Authorized officer |
|---|---|
| **China National Intellectual Property Administration (ISA/ CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2023/091895** |

**C.     DOCUMENTS CONSIDERED TO BE RELEVANT**

| Category* | Citation of document, with indication, where appropriate, of the relevant passages | Relevant to claim No. |
| --- | --- | --- |
| A | CN 112057165 A (SHANGHAI UNITED IMAGING HEALTHCARE CO., LTD.) 11 December 2020 (2020-12-11)<br>        entire document | 1-23 |
| A | CN 113349925 A (ZHEJIANG UNIVERSITY OF TECHNOLOGY) 07 September 2021 (2021-09-07)<br>        entire document | 1-23 |
| A | JP 2007159933 A (HITACHI MEDICAL CORP. et al.) 28 June 2007 (2007-06-28)<br>        entire document | 1-23 |
| A | US 2015283379 A1 (PACESETTER INC.) 08 October 2015 (2015-10-08)<br>        entire document | 1-23 |
| A | WO 2015135058 A1 (SYNAPTIVE MEDICAL BARBADOS INC. et al.) 17 September 2015 (2015-09-17)<br>        entire document | 1-23 |
| A | 张薇等 (ZHANG, Wei et al.). "基于灰度的二维/三维图像配准方法及其在骨科导航手术中的实现 (Implementation of Intensity-based 2D/3D Image Registration in Orthopaedic Navigation System)"<br>中国医学影像技术 (*Chinese Journal of Medical Imaging Technology*),<br>Vol. 23, No. 7, 20 July 2007 (2007-07-20),<br>        entire document | 1-23 |

Form PCT/ISA/210 (second sheet) (July 2022)

EP 4 505 961 A1

**INTERNATIONAL SEARCH REPORT**
**Information on patent family members**

International application No.

**PCT/CN2023/091895**

| Patent document cited in search report | | | Publication date (day/month/year) | Patent family member(s) | | | Publication date (day/month/year) |
|---|---|---|---|---|---|---|---|
| CN | 112163987 | A | 01 January 2021 | None | | | |
| CN | 113057734 | A | 02 July 2021 | None | | | |
| CN | 103479430 | A | 01 January 2014 | None | | | |
| CN | 112057165 | A | 11 December 2020 | None | | | |
| CN | 113349925 | A | 07 September 2021 | None | | | |
| JP | 2007159933 | A | 28 June 2007 | JP | 4915836 | B2 | 11 April 2012 |
| US | 2015283379 | A1 | 08 October 2015 | US | 9999772 | B2 | 19 June 2018 |
| | | | | US | 2018256896 | A1 | 13 September 2018 |
| | | | | US | 10806929 | B2 | 20 October 2020 |
| WO | 2015135058 | A1 | 17 September 2015 | EP | 3116376 | A1 | 18 January 2017 |
| | | | | EP | 3116376 | A4 | 22 November 2017 |
| | | | | EP | 3116376 | B1 | 11 August 2021 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2023091895 W
- CN 202210493274
- CN 202210764281